# EUROPEAN PATENT APPLICATION

(11) **EP 4 335 916 A1**
(43) Date of publication of application: **13.03.2024**
(21) Application number: 22798900.1
(22) Date of filing: 26.04.2022
(51) Int. Cl.: C12N 1/00, C12N 1/14, C12P 7/18, C12P 7/40, C12P 7/50, C12P 7/52, C12P 7/54, C12P 13/00

(54) **FERMENTATION BY MYCORRHIZAL ASCOMYCETOUS WHITE WOOD-ROTTING FUNGI, PRODUCTION OF FERMENTATION PRODUCT FOOD, PROCESSED FOOD, BEVERAGE, TEA, HERBAL MEDICINE, AND LIVESTOCK FEED, METHOD FOR EXTRACTING PHYSIOLOGICALLY ACTIVE SUBSTANCE BY FERMENTATION BY SAID FUNGI, AND METHOD FOR MANUFACTURING PRODUCT OF SAID FUNGI**

(30) Priority: 07.05.2021 JP 2021078944
(71) Applicant: Act LLC, Okinawa 904-0327 (JP); Fujiwara, Sumihisa, Okinawa 904-0327 (JP)
(72) Inventor: UI Seita, Sagae-shi, Yamagata 991-0024 (JP)
(74) Representative: Carridge, Andrew Edward
(86) International application number: PCT/JP2022/018973
(87) International publication number: WO 2022/234794

(57) **Abstract**

An object is to develop a novel fermentation technique by use of fermentation by an ascomycetous white wood-rotting fungus having mycorrhizal characteristics. A physiologically active substance-containing fermented product is manufactured by use of the production of a physiologically active substance by the fermentation of a raw material by an ascomycetous white wood-rotting fungus having mycorrhizal characteristics. Residual agricultural chemicals in a food raw material are degraded by use of the degradation of a persistent compound. A contained component is extracted at a high speed by use of the weakening of a plant cell membrane.

## Description

### Technical Field

The present invention relates to the microbial extraction of a plant physiologically active substance or bioactive substance and the manufacturing of a food, a processed food, a beverage, tea, an herbal medicine, and livestock feed containing a microbial fermentation product.

### Background Art

Since entering the 21st century, the global society has had to solve diverse difficult challenges as if the society must pay a price for having built a rich carbon society by energy and productization based on finite fossil resources of 200 years from the industrial revolution and enjoyed economic development. The global society is finally looking for the decarbonized society or the preservation of the carbon-neutral global environment by around 2050 with the aim of the balance between the environment and economy. This era in the latter part of the 21st century, when seen from another point of view, is the super-aging society and is also the era of a food shortage ascribable to the population projected to increase to 9 billion. If this trend continues, agriculture dependent on agricultural chemicals increasingly progresses for food security under changes in climate. Residual agricultural chemicals will have a more devastating consequence on human bodies, the global environment, and the ecosystem.

The current world comes from frequent natural disasters caused by changes in climate due to global warming ascribable to green gas emission, problems of residual agricultural chemicals on foods brought about by agriculture dependent on agricultural chemicals due to frequent diseases and pests in agricultural crops resulting from rise in temperature, and the amounts of agricultural chemicals used that have increased each year for foods that cover increase in population, and these factors have large influence on the global environment and the ecosystem. Furthermore, science has developed unbiodegradable plastics which ignore nature's law of the earth, and caused CO₂ emission and ocean pollution ascribable to the disposal of industrial waste plastics.

In the global social context, the novel coronavirus suddenly emerged in 2020 and destroyed the economy and the society strenuously built by human beings and the daily life of human beings in a profound manner. Furthermore, coronavirus teaches human beings that strong or weak immunity is a life-and-death matter in the super-aging society. Current medicine has been defeated by the virus. In the meantime, science is trying to counter the virus with vaccines. However, the vaccines are not a technique of eradicating the virus. The vaccines are a technique of constructing a new lifestyle coexisting with the virus. That is, the incoming post-corona society of the 21 century is the "era of immunity" and at the same time, is the era of shift to a sustainable recycling-oriented society from mass production and mass consumption. Agriculture which produces foods, or a food-related industry is not exceptional.

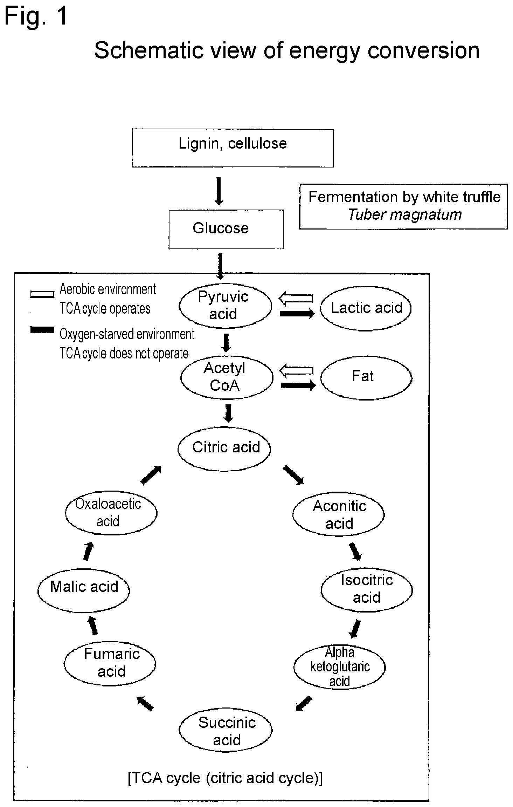

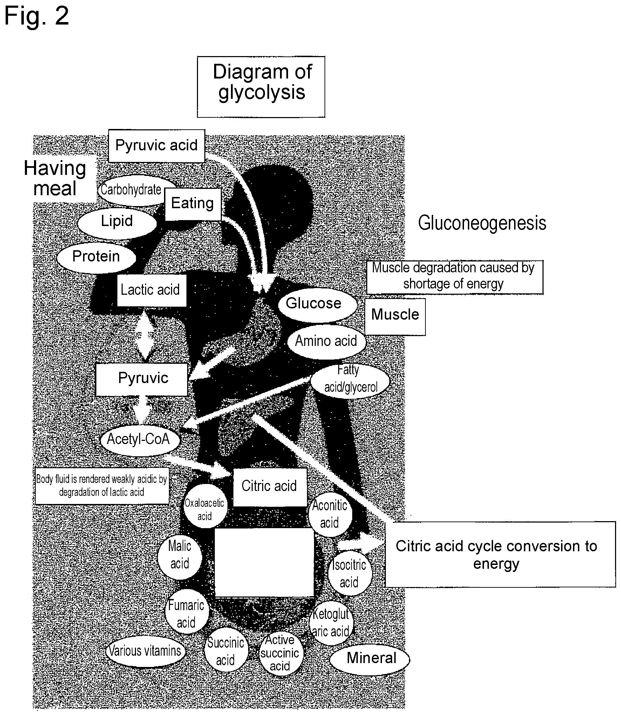

Activity energy in the bodies of human beings which are eukaryotes is obtained from ingested foods, including immunity and antibody production (Figures 1 and 2). Residual agricultural chemicals contained in foods ingested every day are largely related to the immunity. One of the causes of severe symptoms in the elderly is a large number of years for which the elderly has eaten foods contaminated with residual agricultural chemicals. Although the "new lifestyle" in the post-corona society coexisting with the virus has been proposed throughout the world, the most important one is "foods" that influence immune strength. The "new lifestyle" in the super-aging society is that interest in health and immunity must be higher than at present throughout the world. In addition, the development of manufacturing techniques for agricultural chemical-free food production and processed food, beverage, tea, herbal medicine, and livestock feed production is an urgent challenge to the post-corona society, together with measures against changes in climate.

When the present inventor sees, from a higher point of view, current problems faced by the global society and diverse problems and challenges targeted by the global society in 2050, the post-corona or later society is the era of immunity coexisting with viruses, is the super-aging society, the decarbonized society, and a society with the population projected to increase to 10 billion, and at the same time, is the food-insecure society. During such an era, human beings long for long healthy life, whereas immune strength brought about by food is important for a disease-free body, as pointed out by the word "food is medicine", and strong or weak immunity influences health.

One of the factors that reduce immune strength is residual agricultural chemicals in foods. Long heathy life in the new lifestyle is attained by the ingestion of residual agricultural chemical-free foods and the ingestion of immune strength-boosting novel foods, processed foods, beverages, tea, and herbal medicines. This also holds true for livestock. Thus, the present inventor has come up with the idea that it is necessary to find a novel fermenter harmless to humans and animals, to degrade and clean up residual agricultural chemicals by use of this microbe, and to produce and manufacture diverse immune strength-boosting foods, processed foods, beverages, tea, herbal medicines, and livestock feed by such novel fermentation.

Fermented products produced by fermenters currently used widely, such as yeast, koji mold, lactic acid bacteria, *Bacillus subtilis* var. *natto,* and acetic acid bacteria, cannot sufficiently boost immune strength, even if eaten, because these microbes cannot degrade residual agricultural chemicals in raw materials or products. For breaking down such barriers, it is necessary to find a novel fermenter and to develop an unprecedented novel fermented product using this fermenter.

Human beings have created and ingested diverse fermented foods since ancient times because of longing for health. They have used yeast, lactic acid bacteria, Bacillus subtilis var. natto, koji mold, acetic acid bacteria, alcohol yeast, and even *Penicillium.* As for raw materials, they have made use of almost whatever is available. Health food = fermented food is trumpeted in this period. Most of the fermented foods have already been prepared into finished products. However, when the current products are seen from the viewpoint of the future decarbonized society, the disposal of food processing garbage in the era of immunity, immunity, antiaging, and long healthy life, it is necessary to develop a more innovative technique and perform innovation in the most important removal and cleanup of residual agricultural chemicals, products that achieve immune strength boosting by complementing energy, the treatment of processed food residues, and the like.

The present inventor has developed a technique of degrading and cleaning up residual agricultural chemicals in crops or the soil using a mycorrhizal ascomycetous white wood-rotting fungus white truffle *Tuber magnatum,* and previously filed four patent applications (Patent Literatures 1 to 4). Use of this technique enables residual agricultural chemicals to be removed from food, processed food, beverage, tea, feed, and herbal medicine raw materials. A product from which residual agricultural chemicals have been further removed can be manufactured by inserting, to a processing step, the step of dipping a raw material in a white truffle *Tuber magnatum* culture suspension. The preceding finding of the present inventor (Patent Literature 2) is capable of solving the preparation of materials for reduced fertilizers and reduced chemical and chemical-free cultivations by the treatment of food residues. The present inventor has already filed patent applications, as described above, about upstream and downstream techniques of a technique degrading and cleaning up residual agricultural chemicals in a raw material, and a technique of preparing a processing residue into a material for reduced fertilizer and reduced chemical and chemical-free cultivation.

### Citation List

### Patent Literature

Patent Literature 1: Japanese Patent Application No. 2019-164530
Patent Literature 2: Japanese Patent Application No. 2019-164410
Patent Literature 3: Japanese Patent Application No. 2020-142873
Patent Literature 4: Japanese Patent Application No. 2020-216308

### Summary of Invention

### Technical Problem

Thus, problems or challenges to be solved by the present invention are challenges of the following remaining three items.
(1) Development of a technique of producing an immunity-boosting component
(2) Manufacturing throughout the world of diverse foods, processed foods, beverages, tea, herbal medicines, and livestock feed harmless to humans and animals, containing an immunity-boosting component
(3) Short-time and low-cost manufacturing of diverse processed foods, beverages, herbal health drinks, and beverages for livestock by the development of an ultrashort-time technique of extracting a plant physiologically active substance

Objects of the invention of the present inventor are attained by solving these three items. Intended reasons are as follows.

### <Challenge of (1)>

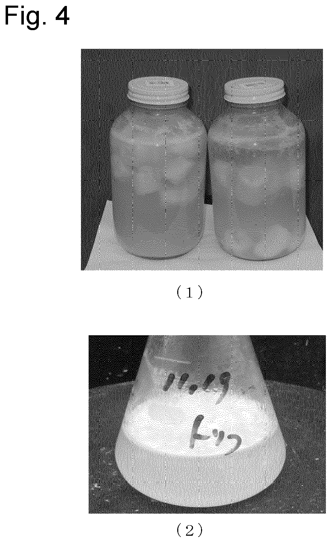

The present inventor has come up with the idea that energy-complementing products adapted to the era of immunity are foods, processed foods, beverages, tea, livestock feed, and herbal medicines containing pyruvic acid which serves as an energy source for every eukaryote of the earth. As mentioned above, microbes producing pyruvic acid have been found among previous fermentative microbes. However, their pyruvic acid is pyruvic acid produced in the middle of the glycolysis pathway from glucose. Therefore, any case has not been found where the pyruvic acid is converted to a subsequent substance in a short time, or bacteria preserve the pyruvic acid in a solid or a liquid without conversion in order to produce the pyruvic acid for their own prey. Accordingly, the present inventor has carried out a test to verify the presence or absence of "fermentation" by white wood-rotting fungi of the family *Pezizaceae* and *Rhizopogonaceae* belonging to mycorrhizal ascomycetous white wood-rotting fungi *Pezizales,* which have not been found in previous fermentation (Figure 4). As a result, the present inventor has found for the first time in the world that white truffle *Tuber magnatum* is a fermenter. Research has been conducted so far on ascomycetous white wood-rotting fungi that produce glucose by degrading lignin or cellulose with an enzyme such as lignin peroxidase, manganese peroxidase, or laccase. Nonetheless, fermentation by white truffle *Tuber magnatum* has not been known. According to this finding, pyruvic acid, a final product of the glycolysis pathway, can be easily produced in a stable manner by fermentation by white truffle *Tuber magnatum* with carbohydrate as a raw material. Furthermore, this test has revealed by analysis that choline chloride and 3-hydroxybutyric acid which are components important for immunity are also produced.

### <Challenge of (2)>

White truffle *Tuber magnatum* is a newly found fermenter, and this fungus produces, in the course of fermentation, pyruvic acid, choline chloride, 3-hydroxybutyric acid, and the like related to the immunity and energy acquirement of humans, animals, and livestock, which cannot be produced by previous fermenters yeast, koji mold, lactic acid bacteria, and *Bacillus subtilis* var. *natto.* An unprecedented novel fermented food, processed food, beverage, tea, herbal medicine, and livestock feed containing the components described above can thereby be manufactured. Theoretically, plant raw materials, fruits, stems, and tuberous roots containing carbohydrate can be used as raw materials in production by fermentation by white truffle *Tuber magnatum.* Therefore, not only crops that are cultivated throughout the world but plants of original species which grow naturally in each country may be used as raw materials. This suggests that the present invention can be carried out throughout the world. Thus, the challenge of (2) is solved.

### <Challenge of (3)>

Many plants contain a physiologically active substance effective for human health. Many products have been developed or commercialized by extracting such a physiologically active substance with water, hot water, an organic solvent, or the like. However, many plant active substances are often insoluble in water, insoluble in hot water, or insoluble in an organic solvent. Thus, all plant physiologically active substances cannot be extracted in a short time by a single extraction step. Hence, unextractable components have not been utilized and have been disposed of as processing wastes without being used.

Fermentation by white truffle *Tuber magnatum* is capable of degrading and weakening lignin and cellulose in cells of plant tissues in a short time owing to the characteristics of white wood-rotting fungi, and thereby rapidly eluting and extracting an intracellular physiologically active substance in a short time at ordinary temperature. A beverage, an herbal health drink, and the like containing the physiologically active substance can be manufactured in a short time by use of such characteristics.

In the preceding application (Patent Literature 1), the present inventor has found for the first time in the world that white truffle *Tuber magnatum,* despite being an ascomycetous mycorrhizal fungus, possesses characteristics capable of degrading lignin and cellulose. The challenge of the present invention is achieved by exploiting such characteristics of white wood-rotting fungi in the present invention.

The present inventor has carried out research tests by setting goals of the following five items.
(1) To produce or manufacture an immune strength-boosting safe and secure food, processed food, beverage, tea, herbal medicine, and livestock feed that contain no or nearly no residual agricultural chemicals and do not influence immunity.

In order to manufacture such a product, cultivation must be performed such that a raw material contains no residual agricultural chemicals. For this purpose, it is desirable to perform reduced fertilizer and reduced chemical and chemical-free cultivation in fields throughout the world to produce residual agricultural chemical-free raw materials. The present inventor has filed four patent applications about this cultivation method, as described above.

However, raw material production is not stably performed unless agriculture dependent on agricultural chemicals must be increasingly performed due to changes in global climate or global warming. Therefore, for a raw material containing residual agricultural chemicals, it is essentially required in the present invention to degrade, detoxify, and clean up residual agricultural chemicals both at cultivation sites and during processing. The present inventor has filed the preceding patent application (Patent Literature 4) about a method for degrading, detoxifying, and cleaning up residual agricultural chemicals in a field crop serving as a raw material by white truffle *Tuber magnatum.* In the present invention, a modification is made to the degradation, detoxification, and cleanup of residual agricultural chemicals in this raw material by dipping in a white truffle *Tuber magnatum* suspension, and a method for manufacturing a safer and more secure product is thereby provided.

(2) To develop unprecedented novel pyruvic acid-containing and choline chloride- and 3-hydroxybutyric acid-containing diverse foods, processed foods, beverages, tea, herbal medicines, and livestock feed by exploiting the fact that the ascomycetous white wood-rotting fungus white truffle *Tuber magnatum* is a fermenter

Pyruvic acid is an energy source for all eukaryotes that inhabit the earth. However, any pyruvic acid-containing product has not been able to be prepared so far. This is because a previous microbe producing pyruvic acid, for example, yeast, produces pyruvic acid from glucose, and then alcohol, while an anaerobic bacterium produces pyruvic acid from glucose, and propagates by preying on this pyruvic acid; thus, pyruvic acid is prey for an own energy source or an intermediate production substance of the glycolysis pathway and is difficult to extract. On the other hand, white truffle *Tuber magnatum* produces glucose from plant lignin or cellulose with a degrading enzyme such as lignin peroxidase, manganese peroxidase, or laccase, and produces pyruvic acid from the glucose by fermentation. White truffle *Tuber magnatum* neither converts the pyruvic acid to alcohol, unlike the yeast, nor preys on the pyruvic acid in itself, unlike the anaerobic bacterium. In the presence of carbohydrate such as lignin or cellulose which is prey for white truffle *Tuber magnatum,* this fungus permanently produces pyruvic acid under conditions where other conditions are satisfied. This pyruvic acid production by fermentation by white truffle *Tuber magnatum* allows for development of globally unprecedented novel pyruvic acid-containing diverse products.

This pyruvic acid is a substance that is converted to energy through a mitochondrial citric acid cycle in allegedly 37 trillion or 60 trillion cells that constitute the human body (Figures 1 and 2). Energy is necessary for sustaining immune strength and for producing many immunocytes. Physical strength for fighting coronavirus is also produced by energy. Its source is pyruvic acid. For the post-corona society, immunity is a key point, and humans' own immune strength rather than vaccines competes with new viruses emerging one after another.

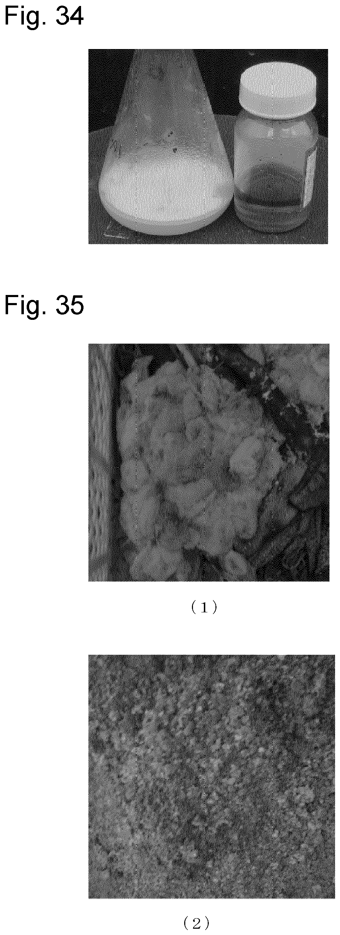

The degradation and cleanup of residual agricultural chemicals by the white truffle *Tuber magnatum* of the present invention, and products such as pyruvic acid-containing foods, processed foods, beverages, tea, and herbal medicines allow for antiaging and long healthy life in the super-aging society. Furthermore, pyruvic acid contained in farming or pet feed attains immunity boosting and livestock or pets that defeat summer fatigue or the like (Figure 35).

(3) To manufacture an herbal medicine component-containing health drink, a pharmaceutical raw material, a skin functional cosmetic raw material, and an agricultural chemical raw material by the short-time extraction of a plant physiologically active substance or a bioactive substance by use of white wood-rotting fungal characteristics of white truffle *Tuber magnatum*

(4) The present invention further provides a food manufacturing technique that conforms with zero CO₂ emission by 2050 and produces no industrial waste, and is capable of reduced fertilizer and reduced chemical and chemical-free cultivation by allowing white truffle *Tuber magnatum* to grow on residues of processed foods, beverages, tea, and the like (Figure 36). The present inventor has previously filed a patent application about this technique (Patent Literature 2).

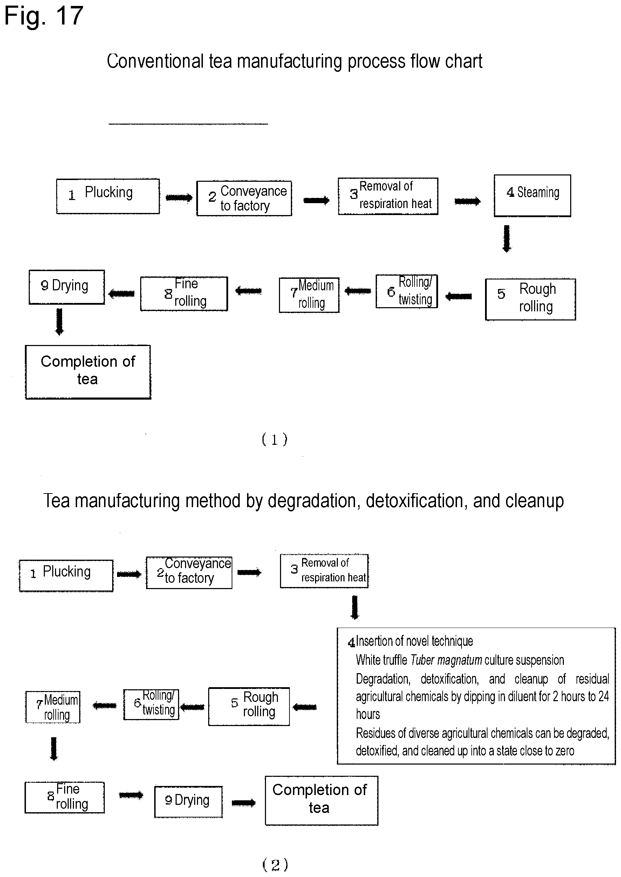

(5) In the present invention, on the assumption that a raw material for a processed food, a beverage, an herbal medicine, or farming feed contains residual agricultural chemicals, a residual agricultural chemical-free product is manufactured by inserting a degradation, detoxification, and cleanup technique using white truffle *Tuber magnatum* to a processing step (Figure 17).

The present inventor has carried out a vast number of tests to solve the challenges described above, and revealed full characteristics of white truffle *Tuber magnatum.* The present invention exploits such diverse characteristics of white truffle *Tuber magnatum.* Particularly, characteristics based on findings about fermentation by white truffle *Tuber magnatum* are important.

Specifically, the present inventor has found that fermentation by the mycorrhizal ascomycetous white wood-rotting fungus white truffle *Tuber magnatum* allows for manufacturing of a next-generation fermented product, which has not been found in previous fermentation. According to this novel finding, pyruvic acid-containing novel diverse functional products adapted to the new lifestyle theme, from which residual agricultural chemicals have been cleaned up can be manufactured by use of some characteristics useful for the present invention among diverse characteristics possessed by white truffle

### Tuber magnatum.

The technique of the present invention has been developed with a view to implementation throughout the world.

The present invention has been achieved by findings of the present inventor about unprecedented fermentation by the mycorrhizal ascomycetous white wood-rotting fungus white truffle *Tuber magnatum* by removing or getting rid of stereotypes on previous fermentation on the basis of enormous findings gained by the tests to elucidate the characteristics of white truffle *Tuber magnatum,* the tests for the four preceding patent applications (Patent Literatures 1 to 4), and additional tests carried out for the present invention.

The post-corona society is the era of immunity, and the present invention employs the novel mycorrhizal ascomycetous wood-rotting fungus white truffle *Tuber magnatum* because not only are the five items mentioned above which are objects of the present invention directed to the manufacturing of novel products such as foods or beverages that achieve immunity boosting, antiaging, or long healthy life, but the product of the present invention enables upstream to downstream techniques to be consistently performed by a single fungus white truffle *Tuber magnatum,* from the degradation, detoxification, and cleanup of residual agricultural chemicals at agricultural field sites of raw material production to diverse processed foods, beverages, herbal medicines, and farming feed containing pyruvic acid which is an energy source for human bodies, and further the treatment of processing residues which are generated in a processing step as well as the production or purveyance of safe and secure processing raw materials that produce no industrial waste owing to the decarbonized society, carbon-neutral zero greenhouse gas emission, and the modification of residues into materials for reduced fertilizers and reduced chemical and chemical-free cultivations by fermentation by white truffle *Tuber magnatum,* because processing raw materials thereof are agricultural products that are produced in agricultural fields worldwide.

Hence, additional tests have been carried out for the present invention on the basis of the vast number of preceding tests related to the present invention as described above to successfully develop diverse innovative novel techniques of the fermentation world by fermentation by white truffle *Tuber magnatum* possessing mycorrhizal ascomycetous white wood-rotting fungal characteristics.

The present inventor has found fermentation by the novel fermenter mycorrhizal ascomycetous white wood-rotting fungus white truffle *Tuber magnatum* (Figure 4). According to this new finding, "fermentation" is imparted to diverse characteristics of white truffle *Tuber magnatum.* An innovative technique for innovation or a product related to processed foods or beverages in the post-corona society and the decarbonized society can thereby be attained. Thus, the present invention has been completed.

Life coexisting with viruses in the post-corona society, foods, processed foods, beverages, tea, and herbal medicines in a new lifestyle, and farming or pet feed are presumed to require higher "health"-conscious products that fit in trends toward immunity, antiaging, and long heathy life than current products. Current fermentation techniques almost run out of new products. Foods fermented by lactic acid bacteria, yeast, *Bacillus subtilis* var. *natto,* and the like are reportedly healthy. However, these microbes cannot completely degrade residual agricultural chemicals. Current diverse products are commercialized and ingested without degrading, detoxifying, and cleaning up residual agricultural chemicals contained in raw materials. As a result, the ingestion of such products might rather have adverse effects. However, it has been impossible to prepare a residual agricultural chemical-free product due to the absence of an effective technique of completely degrading and cleaning up residual agricultural chemicals from raw materials.

Diet and food culture in the new lifestyle in the post-corona society throughout the world must boost immunity. Accordingly, the development of a novel innovative technique of manufacturing a residual agricultural chemical-free product as well as food processing innovation must be performed. This is because, since microbes such as lactic acid bacteria, *Bacillus subtilis* var. *natto,* and yeast are limited by their ability, products beyond the current ones cannot be developed.

In addition to main aspects of the invention, secondarily invented items are listed below.
(i) Manufacturing of a residual agricultural chemical-free food, beverage, feed, and tea, and an immunity-boosting product by the dipping of a processing raw material in a white truffle *Tuber magnatum* suspension.
(ii) Manufacturing of an energy or cell proliferation component (pyruvic acid, choline chloride, 3-hydroxybutyric acid, and indole-3-acetic acid)-containing long healthy life or antiaging product, supplement, processed food, beverage, tea, herbal medicine, livestock or pet feed, and other products fermented by white truffle *Tuber magnatum.*
(iii) Short-time extraction of an active substance or a bioactive substance and manufacturing of an herbal drink.
(iv) Manufacturing of multifunctional bread for immunity boosting and intestinal regulation containing white truffle *Tuber magnatum* mycelium chitin and pyruvic acid.
(v) Manufacturing of an upgrade health food, processed food, beverage, tea, feed, and bread by a composite product of a fermented dairy product and a pyruvic acid-containing product.
(vi) Stable production of a food processing raw material by a material for reduced fertilizer and reduced chemical and chemical-free cultivation of a food or processed food residue.
(vii) A food processing technique that conforms with zero CO₂ emission by 2050 and produces no garbage.
(viii) Hydrogen production by the degradation of pyruvic acid by an anaerobic hydrogen-producing bacterium.
(ix) Production of a pharmaceutical or cosmetic raw material of pyruvic acid, a plant hormone, and indole-3-acetic acid as derivatives from fermentation by white truffle *Tuber magnatum.*
(x) Manufacturing of diverse novel products using agricultural products or special local crops around the world as raw materials.

The food of the present invention means a general food including a staple food, or a cooking ingredient. The processed food of the present invention refers to a food that has undergone some processing, and includes, for example, fish paste products, processed meat products, processed milk products, preferred foods, seasonings, confectionery, frozen foods, retort foods, canned foods, and instant foods. Examples of the beverage of the present invention include tea, green tea, black tea, Chinese tea, oolong tea, senna tea, barley tea, buckwheat tea, blend tea, roasted green tea, Gyokuro (refined green tea), pu-erh tea, Ume-Kombucha (tea made of powdered kelp and pickled plum), mate tea, chocolate, coffee, cocoa, tapioca, refreshing beverages, fruit juice beverages (oranges, apple, grape, etc.), carbonated beverages, functional beverages, sport beverages, and non-alcoholic beverages.

The herbal medicine of the present invention refers to folk medicinal herb or Chinese medicinal herb. The livestock or pet feed of the present invention refers to hay, a cereal residue, or a beverage for livestock or a pet. The physiologically active substance is a physiologically active substance contained in plant cells. Examples thereof include tannin, α-pinene, and polyphenol.

The fermentation product of the present invention means a substance that is produced by a microbe through fermentation. Examples thereof include pyruvic acid, choline chloride, 3-hydroxybutyric acid, indole-3-acetic acid, chitin nanofiber, and fermentation components of white truffle *Tuber magnatum.*

### Solution to Problem

The present invention has been completed by use of characteristics found for the first time by the present inventor through assay tests on diverse characteristics of white truffle *Tuber magnatum* of the family *Tuberaceae* belonging to *Pezizales.* In the present invention, tests were carried out using white truffle *Tuber magnatum.* Approximately 180 *Tuber* spp. to which white truffle *Tuber magnatum* belongs have been found around the world. The order *Pezizales* to which *Tuber* spp. belong also includes fungi possessing mycorrhizal ascomycetous white wood-rotting fungal characteristics, as in white truffle *Tuber magnatum.* Therefore, there is a possibility that these fungal species can be similarly used, depending on future tests or research. Thus, the present invention is not limited by white truffle *Tuber magnatum.*

The characteristics exploited by the present invention are as follows.
1. White truffle *Tuber magnatum* is a fermenter.
2. White truffle *Tuber magnatum* produces pyruvic acid, choline chloride, 3-hydroxybutyric acid, lactic acid, and the like through the degradation and glycolysis of lignin or cellulose.
3. White truffle *Tuber magnatum,* despite being an ascomycetous fungus, possesses white wood-rotting fungal characteristics capable of degrading lignin and cellulose, as in basidiomycetous white wood-rotting fungi.
4. White truffle *Tuber magnatum* produces indole-3-pyruvic acid from pyruvic acid as a raw material, and produces a cell proliferation hormone (plant hormone) indole-3-acetic acid.
5. White truffle *Tuber magnatum* can produce a processed food raw material by rapidly degrading residual agricultural chemicals during raw material cultivation.
6. White truffle *Tuber magnatum* can degrade and clean up residual agricultural chemicals in the step of processing a raw material for processed foods or the like.
7. A physiologically active substance or a bioactive component can be rapidly extracted.
8. White truffle *Tuber magnatum* is a fungus harmless to humans and animals.
9. Its strong antimicrobial activity can inhibit and inactivate pathogenic microbes.
10. White truffle *Tuber magnatum* is a mycorrhizal fungus, and white truffle *Tuber magnatum* is allowed to grow on a food or processed food residue to prepare a soil improver for reduced fertilizer and reduced chemical and chemical-free cultivation, which allows for stable cultivation.
11. White truffle *Tuber magnatum* can degrade a waste plant oil (no "garbage" is produced in the food processing of plants).

The present invention intends implementation throughout the world, and the ascomycetous white wood-rotting fungus white truffle *Tuber magnatum* which has not been utilized so far in the fermentation world has been found to be a fermentative fungus. The present invention has been completed on the basis of this finding.

Since white truffle *Tuber magnatum* is a mycorrhizal fungus, it is difficult to culture new mycelium from fruit body. Hence, mass production of mycelium is very difficult. Therefore, use of white truffle *Tuber magnatum* has not been assumed in the fermentation industry. Hence, the present inventor has had to start tests from the obtainment of the fruit body of white truffle *Tuber magnatum.*

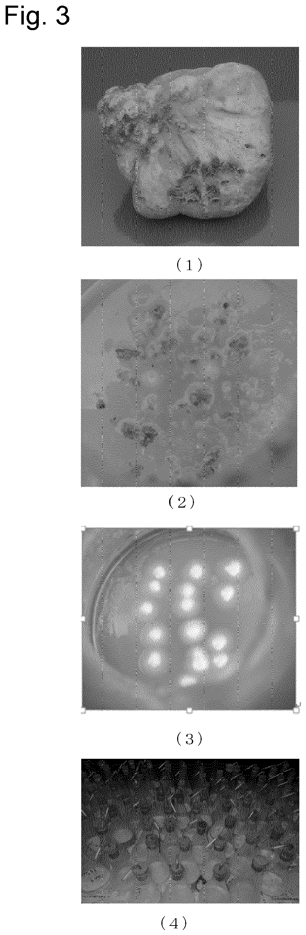

The present inventor has succeeded in mass production of mycelium and an undiluted liquid of white truffle *Tuber magnatum* (Figure 3(4)) using new mycelium obtained by plate culture of a conventional culture technique from white truffle *Tuber magnatum* fruit body (Figure 3(1)) imported from Alba, Italy (Patent Literature 1). Tests were conducted on diverse techniques, products, and product manufacturing related to fermentation by the white truffle fungus suitable for an object of the present invention using the mycelium suspension obtained by this mass culture.

### Advantageous Effects of Invention

Previous fermentative microbes, lactic acid bacteria, yeast, koji mold, or *Bacillus subtilis* var. *natto* cannot degrade a persistent plant compound lignin. These microbes are microbes partly responsible for global carbon circulation, and are incapable of saccharification, glycolysis, and production of "pyruvic acid", "choline chloride", "3-hydroxybutyric acid", and "indole-3-acetic acid", final products of the glycolysis pathway, from carbohydrate by a single microbe.

The present invention exploits fermentation by the mycorrhizal ascomycetous white wood-rotting fungus white truffle *Tuber magnatum* having characteristics of the white wood-rotting fungus, which is only one fungus of the earth that degrades lignin and cellulose, and thereby allows for production of a compound that cannot be manufactured by the previous fermentative microbes, and allows for degradation of residual agricultural chemicals in a fermented product raw material, which cannot be achieved by the previous fermentative microbes.

### Brief Description of Drawings

[Figure 1] Schematic view of energy conversion
[Figure 2] Diagram of glycolysis
[Figure 3] Mass culture of truffle mycelium from fruit body
[Figure 4] Test to verify that white truffle *Tuber magnatum* is fermenter
[Figure 5] Manufacturing of banana pyruvic acid by fermentation by truffle *Tuber magnatum*
[Figure 6] Plant hormone production test by fermentation of processed food residue by white truffle *Tuber magnatum*
[Figure 7] Extraction test of arbutin, novel sourness-masking substance, and novel sweet component by fermentation of pear by white truffle *Tuber magnatum*
[Figure 8] Manufacturing test of banana pyruvic acid-containing dried fruit by white truffle *Tuber magnatum*
[Figure 9] Fermentation and degradation test of glycerin solution by white truffle *Tuber magnatum*
[Figure 10] Degradation test of neonicotinoid by white truffle *Tuber magnatum*
[Figure 11] Herbicide degradation test by degradation by white truffle *Tuber magnatum*
[Figure 12] Herbicide degradation test by white truffle *Tuber magnatum*
[Figure 13] Degradation/cleanup test of residual agricultural chemicals in processing raw material cherry by application of white truffle *Tuber magnatum* suspension
[Figure 14] Degradation and cleanup process flow chart of residual agricultural chemicals in processed food manufacturing process
[Figure 15] Degradation test of residual agricultural chemicals in tea leaf by white truffle *Tuber magnatum*
[Figure 16] Raw material production by novel tea manufacturing method
[Figure 17] Tea manufacturing process flow chart
[Figure 18] Test to verify that white truffle *Tuber magnatum* is fermenter
[Figure 19] Lactic acid production test by fermentation by white truffle *Tuber magnatum*
[Figure 20] Manufacturing test of immune rolled barley by fermentation by white truffle *Tuber magnatum*
[Figure 21] Manufacturing test of immune jam using acidic solution by fermentation by white truffle *Tuber magnatum*
[Figure 22] Short-time manufacturing test of immune fruit juice pyruvic acid beverage by fermentation by white truffle *Tuber magnatum*
[Figure 23] Manufacturing test of pyruvic acid-containing chocolate by fermentation by white truffle *Tuber magnatum*
[Figure 24] Manufacturing test of functional immune bread containing pyruvic acid or mycelium-derived chitin fiber produced by fermentation by white truffle *Tuber magnatum*
[Figure 25] Manufacturing test of pyruvic acid-containing immunity-boosting noodle by fermentation by white truffle *Tuber magnatum*
[Figure 26] Manufacturing test of araliaceous plant green juice by use of fermentation by white truffle *Tuber magnatum*
[Figure 27] Manufacturing test of livestock feed by fermentation by white truffle *Tuber magnatum*
[Figure 28] Manufacturing test of farming feed by fermentation by white truffle *Tuber magnatum*
[Figure 29] Farming feed and chicken egg production test by fermentation by white truffle *Tuber magnatum*
[Figure 30] Short-time extraction test of physiologically active substance or bioactive substance by fermentation by white truffle *Tuber magnatum*
[Figure 31] Persimmon tannin extraction test by fermentation by white truffle *Tuber magnatum*
[Figure 32] Extraction test of wasabi bioactive component allyl isothiocyanate by fermentation by white truffle *Tuber magnatum*
[Figure 33] Pine needle α-pinene extraction test by fermentation by white truffle *Tuber magnatum*
[Figure 34] Degradation test of plant waste oil by fermentation by white truffle *Tuber magnatum*
[Figure 35] Manufacturing of material for reduced fertilizer and reduced chemical and chemical-free cultivation by fermentation of processed food residue by white truffle *Tuber magnatum*
[Figure 36] Plant tissue permeability test of white truffle *Tuber magnatum* culture suspension
[Figure 37] Plant hormone production test by fermentation of processed food residue by white truffle *Tuber magnatum*
[Figure 38] Degradation and cleanup test of honey neonicotinoid by white truffle *Tuber magnatum*

### Description of Embodiments

### Examples

Hereinafter, examples of the present invention will be given. However, the present invention is not limited by these examples.

### [Experimental Example 1]

### <White truffle Tuber magnatum growth test>

White truffle *Tuber magnatum* can be mass-cultured (Figure 3(4)) by culturing hypha elongated from white truffle mycelium of a fruit body lysate (Figure 3(2)) taken out onto the following basal medium, and collecting mycelium from the resulting separated colony (flora) (Figure 3(3)). This flora is composed mainly of chitin.

**[Table 1]**

| [Medium composition and culture conditions for white truffle *Tuber magnatum*] | | |
|---|---|---|
| | Water | 1000 cc |
| | HYPONeX | 3 g |
| | Sugar | 30 g |
| | Agar | 15 g |
| | Lowest temperature | 5°C |
| | Highest | 15°C |
| | Left standing, cultured in bright place | |

The colony thus obtained in a large amount was crushed, and the obtained suspension was used in the following experiments either directly or after being diluted with water.

### [Experimental Example 2]

### <Test 1 to verify that white truffle Tuber magnatum is fermenter>

In order to verify that white truffle *Tuber magnatum* is a fermenter, the following experiment was conducted.

A 2000 cc glass storage jar was filled with 200 g of banana (the skin of which was removed) as a test material and 1800 cc of water, and 20 cc of the white truffle *Tuber magnatum* culture suspension of the present invention was added thereto, followed by static culture in a room of 10°C at the lowest and 25°C at the highest. Since white truffle *Tuber magnatum* possesses strong antimicrobial properties (Patent Literature 1), neither the raw material nor the culture was particularly sterilized.

Figure 4(1) shows a state 7 days after the start of culture. White truffle *Tuber magnatum* was found to actively propagate and perform fermentation. This image verifies that white truffle *Tuber magnatum* is a fermenter.

### [Experimental Example 3]

### <Test 2 to verify that white truffle Tuber magnatum is fermenter>

White truffle *Tuber magnatum* propagates and performs fermentation by preying on sugar. An experiment was conducted on whether white truffle *Tuber magnatum* would also propagate and perform fermentation in glycerin instead of lignin or cellulose.

### [Experimental method]

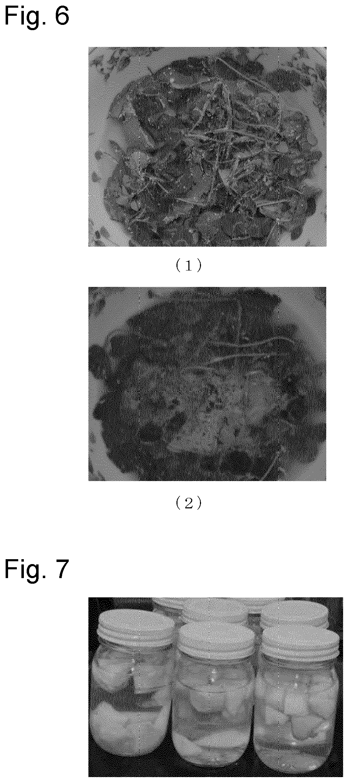

A plant residue was mixed with 1000 cc of water and 30 cc of glycerin, and 20 cc of the white truffle *Tuber magnatum* suspension was added thereto (Figure 6(1)), followed by static culture in a room of 10°C at the lowest and 25°C at the highest.

Figure 6(2) shows a state 10 days after the start of culture. This verified that white truffle *Tuber magnatum,* despite being an ascomycetous wood-rotting fungus, also grows in glycerin instead of lignin or cellulose.

### [Experimental Example 4]

### <Test to verify that pyruvic acid is produced by use of fermentation by white truffle Tuber magnatum>

In order to verify that pyruvic acid can be produced by use of fermentation by white truffle *Tuber magnatum,* the following experiment was conducted.

The white truffle *Tuber magnatum* fermentation medium used contained 1000 cc of water, 3 g of HYPONeX, and 20 cc of a medium fermentation solution of 30 g of molasses (pH 6.0), and culture was performed at a culture temperature from 10°C at the lowest to 25°C at the highest. Figure 4(2) shows a fermented state 7 days later.

Results of analyzing components in this fermentation solution were as follows.

**[Table 2]**

| | Content (% by weight) | |
|---|---|---|
| Pyruvic acid | | 0.6-1.2%. |
| Choline chloride | | 0.01-0.02% |
| Chitin | | 0.003-0.007% |
| Sugar | | 0.3-0.8% |
| Protein | | 0.5-1.5% |
| 3-Hydroxybutyric acid | | 0.001-0.005% |
| Indole-3-acetic acid | | 0.000051-0.00015% |
| Tretinoin | | 0.003-0.009% |
| Acetic acid | | 0.1-0.3% |
| Lactic acid | | 0.003-0.008% |
| Citric acid | | 0.003-0.008% |
| Free amino acid | | Trace |
| Water | | Balance |

### [Experimental Example 5]

### <Test to verify that white truffle Tuber magnatum is fermenter>

In order to verify that white truffle *Tuber magnatum* is a fermenter, the following experiment was conducted.

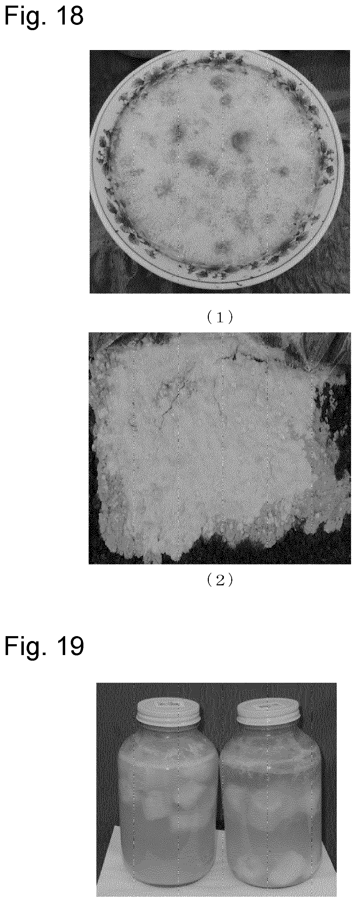

100 g of weeds was administered to 1000 cc of water, and 10 cc of the white truffle *Tuber magnatum* culture suspension was added thereto (Figure 18(1)), followed by static culture in a room under aerobic conditions involving a culture temperature of 15°C at the lowest and 25°C at the highest. Figure 18(2) shows a state 10 days after the start of culture. White truffle *Tuber magnatum* grew and propagated on weeds and actively fermented and degraded the weeds, and thus, white truffle *Tuber magnatum* was found to be a fermenter.

Multifunctional products for immunity boostings, energy replenishment, antiagings, isotonic drinks, and long heathy lives can be manufactured by use of this.

Accordingly, components produced by fermentation by white truffle *Tuber magnatum* using HYPONeX medium were analyzed to examine what components were produced.

Culture solution composition for fermentation was 1000 cc of water, 3 g of HYPONeX (NPK source), and 30 g of white sugar, and white truffle *Tuber magnatum* was added thereto, followed by culture at a temperature from 15°C at the lowest to 30°C at the highest. A fermentation solution 15 days later was analyzed. The results are shown below.

**[Table 3]**

| | Content (% by weight) | |
|---|---|---|
| Pyruvic acid | | 0.6-1.2%. |
| Choline chloride | | 0.01-0.02% |
| Chitin | | 0.003-0.007% |
| Sugar | | 0.3-0.8% |
| Protein | | 0.5-1.5% |
| 3-Hydroxybutyric acid | | 0.001-0.005% |
| Indole-3-acetic acid | | 0.000051-0.00015% |
| Tretinoin | | 0.003-0.009% |
| Acetic acid | | 0.1-0.3% |
| Lactic acid | | 0.003-0.008% |
| Citric acid | | 0.003-0.008% |
| Free amino acid | | Trace |
| Water | | Balance |

The results verified that fermentation by white truffle *Tuber magnatum* ferments and produces components related to immunity or cell proliferation, such as pyruvic acid, choline chloride, 3-hydroxybutyric acid, and indole-3-acetic acid, which cannot be produced by previous fermentative microbes. Multifunctional products for immunity boostings, energy replenishment, antiagings, isotonic drinks, and long heathy lives can be manufactured by adding the components thus produced to diverse products.

### [Experimental Example 6]

### <Test to verify that lactic acid is produced by use of fermentation by white truffle Tuber magnatum>

In order to verify that lactic acid can be produced by use of fermentation by white truffle *Tuber magnatum,* the following experiment was conducted.

A container was filled with the white truffle *Tuber magnatum* fermentation medium containing 1000 cc of water and 200 g of peeled banana, and 10 cc of white truffle *Tuber magnatum* was added thereto, followed by static culture in a room of ordinary temperature at a temperature from 15°C at the lowest to 25°C at the highest. A fermentation solution 72 hours later (Figure 19) was filtered, and components in the solution were analyzed. The results are shown below.

**[Table 4]**

| | Content (% by weight) | |
|---|---|---|
| Mannose | | 0.5-1.5 |
| Butanediol | | 0.1-0.3 |
| Sugar | | 0.4-0.7 |
| Lactic acid | | 0.2-0.5 |
| Acetic acid | | 0.1-0.2 |
| Protein | | 0.05-0.10 |
| Sodium gluconate | | 0.03-0.08 |
| Citric acid | | 0.001-0.004 |
| Water | | Balance |

This beverage was analyzed at "Japan Food Research Laboratories". Data based on the Food Labeling Standards was as follows.

**[Table 5]**

| Analysis test results | | | | |
|---|---|---|---|---|
| Analysis test item | Results | Quantification lower limit | Remarks | Method |
| Moisture | 99.0 g/100 g | - | | Drying method by heating under reduced pressure |
| Protein | Less than 0.1 g/100 g | - | 1 | Combustion method |
| Lipid | Less than 0.1 g/100 g | - | | Soxhlet extraction method |
| Ash | Less than 0.1 g/100 g | - | | Direct ashing method |
| Carbohydrate | 1.0 g/100 g | - | 2 | - |
| Glucide | 1.0 g/100 g | - | 3 | - |
| Dietary fiber | Less than 0.1 g/100 g | - | | Enzymatic-gravimetric method |
| Energy | 4 kcal/100 g | - | 4 | - |
| Sodium | 1.3 mg/100 g | - | | Atomic absorption photometry |
| Sodium chloride equivalent | 0.0033 g/100 g | - | 5 | - |

| | | | | |
|---|---|---|---|---|
| Remark 1. Nitrogen-to-protein conversion factor: 6.25 Remark 2. Calculation expression based on the Food Labeling Standards (Cabinet Office Ordinance No. 10 of 2015): 100-(Moisture + Protein + Lipid + Ash) Remark 3. Calculation expression based on the Food Labeling Standards (Cabinet Office Ordinance No. 10 of 2015): 100-(Moisture + Protein + Lipid + Ash + Dietary fiber) Remark 4. Energy conversion factor based on the Food Labeling Standards (Cabinet Office Ordinance No. 10 of 2015): Protein, 4; Lipid, 9; Glucide, 4; Dietary fiber, 2 Remark 5. Calculation expression: Sodium × 2.54 | | | | |

This test verified that fermentation by white truffle *Tuber magnatum* produces "lactic acid" by fermentation for a short period of 72 hours through the glycolysis pathway of carbohydrate contained in banana. In preceding findings, microbes producing lactic acid from sugar have been regarded as "lactic acid bacteria". However, this test revealed that white truffle *Tuber magnatum,* which is an ascomycetous white wood-rotting fungus, produces "lactic acid" in the glycolysis pathway of fermentation.

Pyruvic acid, a final product of the glycolysis pathway, is produced from this lactic acid by continuing fermentation. An object of the present invention is to manufacture diverse pyruvic acid-containing immunity-boosting foods, processed foods, beverages, and herbal medicines by fermentation by white truffle *Tuber magnatum.* This test revealed that the glycolysis of carbohydrate -> glucose -> lactic acid -> pyruvic acid is performed by a single fungus of white truffle *Tuber magnatum.* It can thus be expected that diverse non-alcoholic products are manufactured by fermentation by white truffle *Tuber magnatum* for a short period.

Butanediol detected in this experiment is produced as a general-purpose raw material for diverse chemical products in petroleum chemistry, and is a compound that has received attention because its microbial production has been studied in recent years.

### [Experimental Example 7]

### <Manufacturing test of immune drink extracted from grape in short time by fermentation by white truffle Tuber magnatum>

This test was ultrarapid manufacturing of pyruvic acid-containing immunity-boosting non-alcoholic grape juice by novel fermentation by white truffle *Tuber magnatum.*

### [Manufacturing method]

A 500 cc PET bottle was filled with 450 cc of water, 50 g of unpeeled grape (cultivar: Delaware), and 10 cc of the white truffle *Tuber magnatum* suspension, followed by static culture at a culture temperature from 20°C at the lowest to 35°C at the highest for 72 hours. As a result, excellently smooth pyruvic acid-containing grapefruit juice was able to be manufactured.

This beverage was analyzed at "Japan Food Research Laboratories". Data based on the Food Labeling Standards was as follows.

**[Table 6]**

| Analysis test results | | | | |
|---|---|---|---|---|
| Analysis test item | Results | Quantification lower limit | Remarks | Method |
| Moisture | 98.2 g/100 g | - | | Drying method by heating under reduced pressure |
| Protein | Less than 0.1 g/100 g | - | 1 | Combustion method |
| Lipid | Less than 0.1 g/100 g | - | | Soxhlet extraction method |
| Ash | Less than 0.1 g/100 g | - | | Direct ashing method |
| Carbohydrate | 1.8 g/100 g | - | 2 | - |
| Glucide | 1.8 g/100 g | - | 3 | - |
| Dietary fiber | Less than 0.1 g/100 g | - | | Enzymatic-gravimetric method |
| Energy | 7 kca1/100 g | - | 4 | - |
| Sodium | 1.5 mg/100 g | - | | Atomic absorption photometry |
| Sodium chloride equivalent | 0.0038 g/100 g | - | 5 | - |

| | | | | |
|---|---|---|---|---|
| Remark 1. Nitrogen-to-protein conversion factor: 6.25 Remark 2. Calculation expression based on the Food Labeling Standards (Cabinet Office Ordinance No. 10 of 2015): 100-(Moisture + Protein + Lipid + Ash) Remark 3. Calculation expression based on the Food Labeling Standards (Cabinet Office Ordinance No. 10 of 2015): 100-(Moisture + Protein + Lipid + Ash + Dietary fiber) Remark 4. Energy conversion factor based on the Food Labeling Standards (Cabinet Office Ordinance No. 10 of 2015): Protein, 4; Lipid, 9; Glucide, 4; Dietary fiber, 2 Remark 5. Calculation expression: Sodium × 2.54 | | | | |

### [Experimental Example 8]

### <Manufacturing test of banana pyruvic acid-containing beverage by use of white truffle Tuber magnatum>

A manufacturing experiment of a banana pyruvic acid-containing beverage was conducted by use of fermentation by white truffle *Tuber magnatum.*

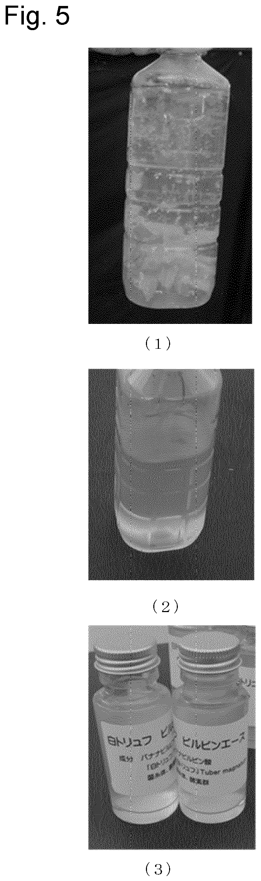

A glass storage jar heat-sterilized at 100°C for 10 minutes was filled with two bananas (the skin of which was removed) as a test material and 2000 cc of water, and 10 cc of the white truffle *Tuber magnatum* culture suspension of the present invention was added thereto, followed by static culture in a room of 10°C at the lowest and 25°C at the highest for 3 to 5 days (Figure 5(1)).

Figure 5(2) shows a solution obtained by filtering a sufficiently fermented solution.

Banana contains starch, carbohydrate, glucide, protein, free amino acids, and the like necessary for fermentation. White truffle *Tuber magnatum* degrades and ferments these components to produce pyruvic acid. A pyruvic acid-containing immune functional drink that offers long heathy life, ameliorates disease or debilitation, and has a very high antiaging effect is manufactured by mixing this fermentation solution with livestock feed, and thereby complementing an energy source. This solution can also be used in beverages for humans, and its daily intake can be approximately 40 cc per 50 kg of body weight. Figure 5(3) shows a prototype.

Also, a beverage that possesses an immunity-boosting function can be easily manufactured by appropriately adding the banana pyruvic acid-containing solution fermented by white truffle *Tuber magnatum,* which is manufactured by this method, to a liquid extracted from a green tea or black tea raw material with water or hot water, or a tapioca drink or the like recently very popular as a preferred beverage.

### [Experimental Example 9]

### <Extraction test of arbutin, novel sourness-masking substance, and novel sweet component by fermentation of pear using white truffle Tuber magnatum>

Pear such as La France contains a functional skin-whitening component arbutin as well as a sourness-masking substance and a carb-free sweet substance newly found by the present invention. An ultrarapid extraction test of these components was conducted by fermentation by white truffle *Tuber magnatum.*

The pear La France was used as a test material, and a 500 cc storage jar was filled with 500 cc of water and 100 g of unpeeled pear slices, followed by static culture in a bright place in a room at a culture temperature from 10°C at the lowest to 25°C at the highest.

Figure 7 shows state 3 days after the start of culture. As is evident from the photograph, intracellular components become easy to extract because white truffle *Tuber magnatum* ultrarapidly degrades and weakens lignin and cellulose in the skin and cells of La France. It can thus be expected that effective trace components in the La France are extracted in a short time.

### [Experimental Example 10]

### <Manufacturing test of banana pyruvic acid-containing dried fruit by use of white truffle Tuber magnatum>

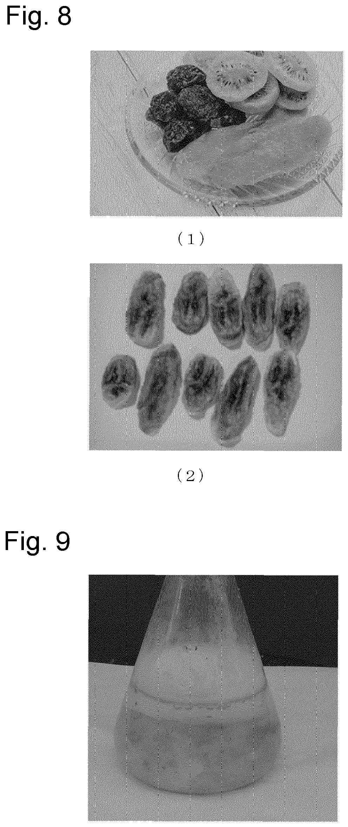

Although diverse dried fruits are commercially available as processed foods of fruits (Figure 8(1)), previous dried fruits have been prepared by drying for the purpose of preservation.

The dried fruit according to the present invention can be manufactured as an immune functional dried fruit containing components such as pyruvic acid because fermentation by white truffle *Tuber magnatum* is performed.

In Figure 8(2), banana is used as a test material, and peeled banana is dipped in water. The height of the water is set to the height of the banana, and the white truffle *Tuber magnatum* culture solution is added thereto. The banana is cultured at a temperature of 10°C at the lowest and 25°C at the highest for 3 to 5 days, and heat-dried when white truffle *Tuber magnatum* propagates on the banana. Most fruits can be prepared into immune dried fruits by the method described above.

Also, powders may be manufactured.

### [Experimental Example 11]

### <Fermentation and degradation test of glycerin solution by white truffle Tuber magnatum>

Waste oils of plant oils are currently recycled as bio oils which are fuels for diesel vehicles. "Glycerin" and "glycerin-containing treated water" are generated in the course of this recycling, and a great deal of cost is required for this treatment. If diesel vehicles will be switched to electric vehicles and be no longer in production in the near future, enormous amounts of waste oils of edible oils currently used in food processing will become new industrial wastes and become waste fats and oils with no destination.

One of the objects of the present invention is to degrade such a food processing waste fat and oil which will become problematic in the near future.

In a testing method, 3 g of HYPONeX (containing N:6.5% P:6% K:19%) and 10 cc of the white truffle *Tuber magnatum* suspension were added to 980 cc of water and 20 cc of glycerin (2% glycerin solution), and this mixed solution was dispensed to 300 cc Erlenmeyer flasks, followed by static culture in a room at a culture temperature from 5°C at the lowest to 15°C at the highest. Components in the treated water thus obtained were as follows.

**[Table 7]**

| | Content (% by weight) | |
|---|---|---|
| 1,3-Butanediol | | 0.2-0.4 |
| Glycerol | | 0.1-0.2 |
| Butyric acid | | 0.05-0.1 |
| Protein | | 0.03-0.07 |
| Sugar | | 0.01-0.04 |
| Protein | | 0.05-0.10 |
| Lactic acid | | 0.005-0.020 |
| Acetic acid | | 0.003-0.008 |
| Citric acid | | 0.0003-0.0007 |
| Water | | Balance |

Figure 9 shows a state fermented by white truffle *Tuber magnatum* 7 days after the start of culture. This verified that white truffle *Tuber magnatum* grows and ferments a glycerin solution at a low temperature in a short time. This test suggests that a food waste edible fat and oil can be treated with white truffle *Tuber magnatum.*

### [Experimental Example 12]

### <Verification test of degradation of agricultural chemicals by white truffle Tuber magnatum>

The most problematic global agricultural chemicals among residual agricultural chemicals in the organisms' ecosystem are "neonicotinoid agents". These agricultural chemicals possess characteristics excellent in residual effectiveness and as such, are often used in insect pest control worldwide. Because of large influence of residual components of the agricultural chemicals on diverse insects' ecosystem, particularly, honeybee, ban on the use thereof is expanding around the world.

Thus, a degradation and detoxification test of residual agricultural chemicals was conducted by the application of a dilution of the "white truffle" *Tuber magnatum* suspension using neonicotinoid-based agricultural chemicals "nitenpyram".

In order to verify that the insecticidal neonicotinoid agent (nitenpyram) is degraded by use of white truffle *Tuber magnatum,* the following test was conducted.
Test material: two trays of mycorrhizal *Chrysanthemum pacificum Nakai*
Name of agricultural chemicals: 100-fold dilution of nitenpyram
Agent degrading and detoxifying residual agricultural chemicals: 100-fold solution of the white truffle *Tuber magnatum* suspension

The test was carried out twice (Example A and Example B).

### Experiment A

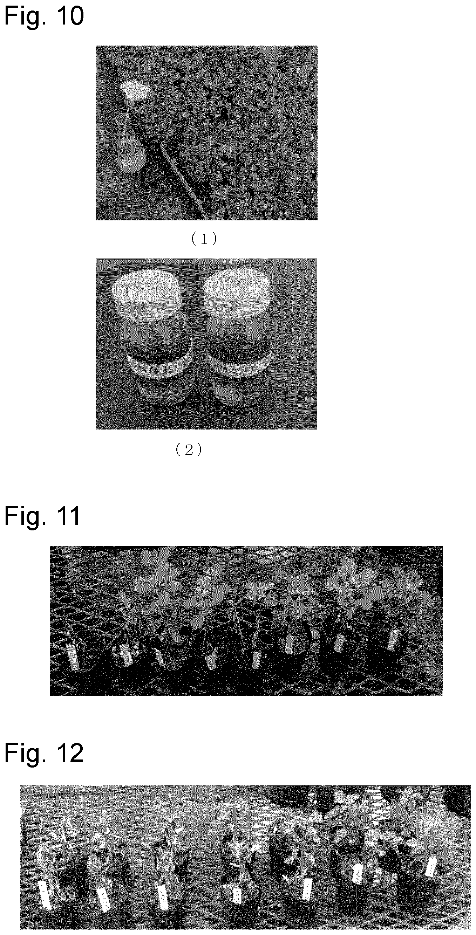

(1) A 1000-fold dilution of nitenpyram was spray-applied to two trays of mycorrhizal *Chrysanthemum pacificum Nakai* (Figure 10(1)).
(2) 24 hours after application of nitenpyram to one of the trays, the white truffle *Tuber magnatum* suspension was applied thereto (treated area), while the remaining one tray was used as an untreated area.
(3) Stems and leaves of mycorrhizal *Chrysanthemum pacificum Nakai* were collected from each of the trays of the untreated area and the treated area 3 days later and 7 days later, and the concentration of residual agricultural chemicals after application of nitenpyram and the concentration of residual agricultural chemicals of nitenpyram 24 hours and 5 days after application of the white truffle *Tuber magnatum* suspension were assayed.

For the preparation of specimens, 10 g of mycorrhizal *Chrysanthemum pacificum Nakai* was collected from each of the treated area and the untreated area, and ground in a mortar, and 1 g of the resulting specimen was mixed with 99 cc of honey to prepare 100 g of a specimen solution (honey of 100-fold diluted mycorrhizal *Chrysanthemum pacificum Nakai*) (HG1 in Figure 10(2)).

### Experiment B

The degradation of agricultural chemicals progresses gradually after application even if spontaneously left. Therefore, in order to verify the effect of application of the white truffle *Tuber magnatum* suspension, the same experiment as experiment A was conducted except that the white truffle *Tuber magnatum* suspension was applied 48 hours later. Mycorrhizal *Chrysanthemum pacificum Nakai* was collected from the untreated area and the white truffle *Tuber magnatum* suspension-treated area on the same day 6 days after application of nitenpyram, and ground. Then, 1 g thereof was mixed with 99 g of honey to prepare 100 g each of specimens (honey of 100-fold diluted mycorrhizal *Chrysanthemum pacificum Nakai*), which was then assayed (MM2 in Figure 10(2)).

As for the specimens of experiments A and B, an analytics company (Mie Prefecture Environmental Conservation Agency) was requested to assay components of their residual agricultural chemicals.

### Assay results

Experiment A Level of components of nitenpyram residual agricultural chemicals 5 days after application of the white truffle *Tuber magnatum* suspension
Untreated area: 0.52 ppm
Treated area: 0.01 ppm (measurable limit of the analyzer)

The residual components 1 day after application of the nitenpyram insecticide had a numeric value as very high as 0.52 ppm, whereas the nitenpyram residual components 5 days after application of the white truffle *Tuber magnatum* suspension were degraded and detoxified into 0.01 which was nearly zero. The value of environmental standards of the nitenpyram residual components is 0.01 ppm.

Experiment B Level of components of nitenpyram residual agricultural chemicals 6 days after application of the white truffle *Tuber magnatum* suspension
Untreated area: 0.03 ppm
Treated area: 0.01 ppm (measurable limit of the analyzer)

As shown in the results of experiment A, the residual components were of 0.52 ppm on the day following application in the untreated area, which was decreased to 0.03 ppm by spontaneous degradation. On the other hand, the level was decreased to 0.01 ppm which was nearly zero in the white truffle *Tuber magnatum* suspension-treated area (application 48 hours later).

Experiments A and B verified that the application of the white truffle *Tuber magnatum* suspension degrades and detoxifies nitenpyram residual agricultural chemicals into the measurable limit 0.01 ppm of analysis.

As shown in the assay values described above, the experiments verified that the spray application of the white truffle *Tuber magnatum* suspension 24 hours and 48 hours after application of agricultural chemicals degrades and detoxifies components of residual agricultural chemicals *in vivo* in crops or plants.

Any technique of degrading and detoxifying *in vivo* residual agricultural chemicals in the application of agricultural chemicals during plant or crop growth is found in neither preceding findings nor preceding literatures. Thus, this test is based on "white truffle" *Tuber magnatum* according to a novel idea.

The numeric values brought about by this assay suggest that the spray application of the 100-fold dilution of the "white truffle" *Tuber magnatum* suspension 24 hours to 48 hours after application of agricultural chemicals degrades and detoxifies components of residual agricultural chemicals *in vivo* in crops during growth into a low-concentration area that is undetectable in analyzers.

This finding suggests that the method of the present invention can degrade and detoxify *in vivo* components of residual agricultural chemicals ascribable to the application of agricultural chemicals during growth. Thus, this novel technique can solve problems of global environment and residual agricultural chemicals, which are critical challenges, including not only food safety but the health of agricultural workers and influence on the organisms' ecosystem in field areas, by future widespread use of this technique all over the world.

Furthermore, the white truffle *Tuber magnatum* suspension was mixed and added at 10% to honey containing a trace amount of nitenpyram, and the mixture was left for 30 days and analyzed for components. As a result, nitenpyram was decreased to 0.01 ppm in both experiments A and B, though being 0.03 ppm in untreated honey.

This test verified that white truffle *Tuber magnatum* degrades and cleans up a neonicotinoid agent which has serious influence on the honeybee's ecosystem due to residual agricultural chemicals. The fermentation treatment by white truffle *Tuber magnatum* of the present invention can be expected to reduce residual agricultural chemicals contained in a trace amount in a raw material, in a fermented product, and enables a residual agricultural chemical-free food, processed food, beverage, herbal medicine, tea, and livestock feed to be manufactured.

### [Experimental Example 13]

### <Verification test of degradation and detoxification of herbicide by white truffle Tuber magnatum>

In order to verify that an anticrop herbicide that has the strongest toxicity among agricultural chemicals and causes crop injury is degraded and detoxified by use of white truffle *Tuber magnatum,* the following experiment was conducted using herbicides "Roundup", "2,4-D", "MCPP", "Catholon" "Tebuthiuron" "Basta", and "Kusatoroze" (all are trade names of herbicides) as test materials.
(1) Two mycorrhizal *Chrysanthemum pacificum Nakai* pots (9 cm) were provided per herbicide.
(2) A reference dilution of each herbicide was prepared, and sprayed to stems and leaves of mycorrhizal *Chrysanthemum pacificum Nakai* in one of the pots until sufficiently wet (approximately 5 to 10 times the amount of the herbicide used in an actual field), and the remaining one pot was used as an untreated area.
(3) A 100-fold solution of the white truffle *Tuber magnatum* suspension was applied to each mycorrhizal *Chrysanthemum pacificum Nakai* pot except for the untreated area 24 hours after application of the herbicide.
(4) A state 60 days after application of the white truffle *Tuber magnatum* suspension was observed (Figure 11) .

In the photograph (Figure 11), "Tebuthiuron", "MCPP", "2,4-D", "Kusatoroze", "Roundup", "Catholon", "Basta", and "untreated area" are shown in order from the left. From this test, it can be visually confirmed that the application of the "white truffle" *Tuber magnatum* suspension degrades and detoxifies residual agricultural chemicals *in vivo* in crops during growth.

### [Experimental Example 14]

### <Detoxification test of Roundup by 30-fold dilution of white truffle Tuber magnatum suspension>

An object of the present invention is to manufacture an unprecedented product using an agricultural chemical-free raw material in a food, a processed food, a beverage, tea, an herbal medicine, or livestock feed. Current product raw materials contain residual agricultural chemicals of herbicides, which weaken the immunity of human bodies, livestock, and pets. For the product described above in the post-corona society, it is desirable to develop, unlike previous products, a novel product that emphasizes immunity. Accordingly, this test was conducted.

In the present invention, the "white truffle" *Tuber magnatum* suspension is applied after application of an herbicide to degrade and detoxify residual components in the soil or the ecosystem and clean up the environment. By contrast, most of the herbicides currently used act on the physiologically active functions of plants so that the plants can no longer survive and are thus killed. *In vivo* residual components of a germicide or an insecticide can be degraded if an enzyme group of "white truffle" *Tuber magnatum* degrades *in vivo* herbicide components before an herbicide applied to leaves is absorbed to cells and migrated to the whole plant tissues so as to act on physiologically active substance-producing functions.

In order to examine how many hours are required for most effectively degrading components of agricultural chemicals by the application of the "white truffle" Tuber *magnatum* suspension after application of an herbicide, the following test was conducted.

### [Testing method]

The test agricultural chemicals used were Roundup (Max Load), and a solution supplemented with 100 cc of Roundup per 1000 cc was spray-applied on leaves in 14 mycorrhizal *Chrysanthemum pacificum Nakai* pots (a solution with a concentration 10 times higher a usual application concentration was applied).

A 100-fold dilution of the "white truffle" *Tuber magnatum* suspension was applied as follows.

A area: untreated, B area: 4 hours later, C area: 6 hours later, D area: 12 hours later, E area: 24 hours later, F area: 60 hours later, and G area: 72 hours later were provided in order from the left, and two pots were set for each area. The white truffle *Tuber magnatum* suspension was sprayed after a lapse of the times described above from application of Roundup, and the subsequent state of mycorrhizal *Chrysanthemum pacificum Nakai* was continuously observed to thereby conduct a test to verify the optimum application time for Roundup degradation and detoxification.

Figure 12 (photograph) is an image for observing the degree of degradation of *in vivo* residual components in the application of Roundup by the application of the "white truffle" *Tuber magnatum* suspension on each elapsed time. A area: untreated, B area: 4 hours later, C area: 6 hours later, D area: 12 hours later, E area: 24 hours later, F area: 60 hours later, and G area: 72 hours later are shown in order from the left, and the image was obtained 30 days after treatment.

### [Results]

### Discussion

*In vivo* Roundup residual components were unable to be degraded and detoxified in the untreated area and the application of the white truffle *Tuber magnatum* suspension 4 hours and 6 hours after application of Roundup. Therefore, both the two pots were killed 30 days after treatment. In the treated area 12 hours later, one pot died, and the other pot survived. In the application areas 24 hours later, 48 hours later, 60 hours later, and 72 hours later, "white truffle" *Tuber magnatum* degraded and detoxified residual components that penetrated the body, whereby mycorrhizal *Chrysanthemum pacificum Nakai* survived. Particularly, the growth of *Chrysanthemum pacificum Nakai* was favorable 24 hours later and 48 hours later, demonstrating that degradation and detoxification treatment 24 hours to 48 hours after application of agricultural chemicals is desirable for the degradation of *in vivo* residual agricultural chemicals in crops by the application of a 100-fold dilution of the white truffle *Tuber magnatum* suspension on leaves.

This test revealed that the application of a 100-fold dilution of the "white truffle *"Tuber magnatum* suspension on leaves 24 hours to 60 hours after application of agricultural chemicals efficiently degrades and detoxifies components of residual agricultural chemicals in crop bodies.

### [Experimental Example 15]

### <Degradation and cleanup test of residual agricultural chemicals in processing raw material cherry by white truffle Tuber magnatum>

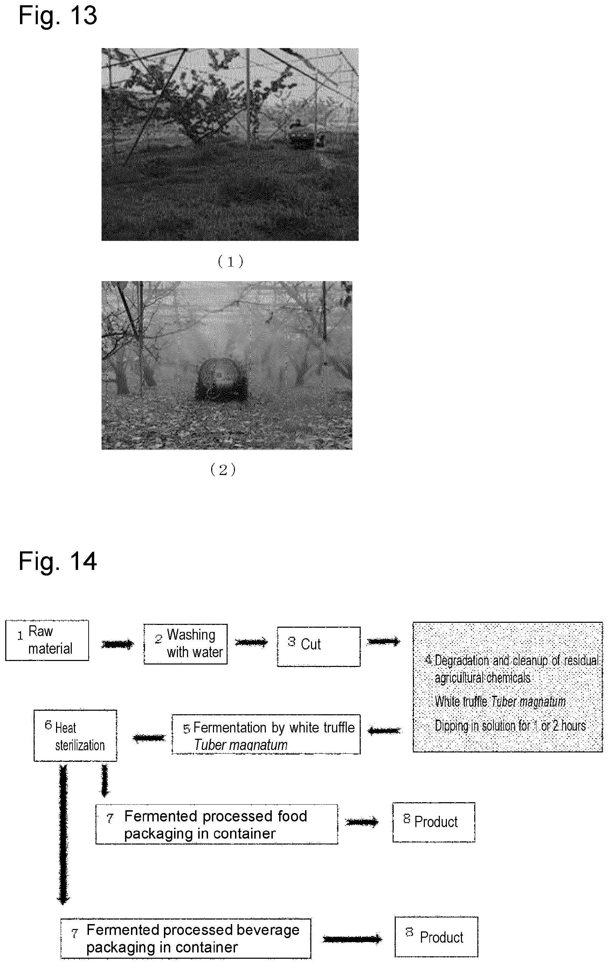

The application of agricultural chemicals to cherry in Yamagata, Japan involves, as shown in Figure 13, applying the agricultural chemicals as scheduled in the calendar of application of agricultural chemicals provided by the prefecture. Cherry is used as a raw material for processed foods and however, contains residual agricultural chemicals, albeit below the national standard, because the agricultural chemicals are applied in such a large amount.

Accordingly, a germicide or an insecticide was applied, and several days after its effect was exerted, a 30-fold dilution of the white truffle *Tuber magnatum* suspension was applied with the same apparatus thereas. Harvested cherry was examined for residual agricultural chemicals.

### Testing method

(1) The application of agricultural chemicals was carried out for approximately 3 months in accordance with the Yamagata standard for use of agricultural chemicals.
(2) The 30-fold dilution of the white truffle *Tuber magnatum* suspension was applied with the same apparatus as that for the application of the agricultural chemicals 5 days after final application of the agricultural chemicals.
(3) Cherry was collected 5 days after application of the white truffle *Tuber magnatum* suspension and sent to an analytics company (Tsukuba Analysis Center Co., Ltd.), which was requested to analyze it.

The analysis was carried out for all of 250 target agricultural chemicals based on "Analytical Methods for Residual Compositional Substances of Agricultural Chemicals, Feed Additives, and Veterinary Drugs in Food" in Notice Syoku-An No. 0124001, dated January 24, 2005. As a result, the detected ones were only 3 types:
Clothianidin: 0.1 ppm (4.0 ppm)
Tebuconazole: 0.6 ppm (5.0 ppm)
Permethrin: 0.1 ppm (5.0 ppm),
all of which were below standard values, while the other components were not detected. The numeric values within the parentheses are the standard values.

In the case of manufacturing various fermented foods or drinks by use of white truffle *Tuber magnatum,* residual agricultural chemical-free food or drink products can be manufactured by degrading and cleaning up residual agricultural chemicals in a processing raw material.

Specifically, a raw material is washed with water and cut. At this stage, the raw material is dipped in a white truffle *Tuber* magnatum-containing solution for 1 to 2 hours to thereby degrade residual agricultural chemicals in the raw material. Then, fermentation treatment using white truffle *Tuber magnatum* is newly performed. After the completion of fermentation, the resultant is heat-sterilized and packaged as a product in a container or the like (Figure 14).

### [Experimental Example 16]

### <Raw material preparation test by novel tea manufacturing method using white truffle Tuber magnatum>

A current tea manufacturing method performs the degradation, detoxification, and cleanup of residual agricultural chemicals neither in a field nor in the process of processing of tea manufacturing. This is because the current tea manufacturing method holds from the viewpoint that agricultural chemicals applied in a field are spontaneously degraded. Tea drinking is often performed because it is good for health. Nonetheless, if the tea leaves contain residual agricultural chemicals, tea drinking also causes ingestion of extracted and melted residual agricultural chemicals because a physiologically active substance or a bioactive substance of the tea leaves is extracted with water or hot water and drunk. Tea which is drunk in consideration of health might become tea that rather weakens "immunity".

Tea in the incoming new era of immunity should be tea from which residual agricultural chemicals have been cleaned up. The present inventor developed the following novel tea manufacturing method from the viewpoint described above.

Specifically, (i) the method involves applying, to field tea leaves, a mixed solution of a 50-fold dilution of the white truffle *Tuber magnatum* suspension supplemented with agricultural chemicals in a prescribed threshold quantity, and manufacturing Sencha (steeped tea) by a conventional method from the tea leaves with reduced residual agricultural chemicals in the raw material tea leaves. Accordingly, how much residual agricultural chemicals would be degraded by the novel tea manufacturing method was verified.

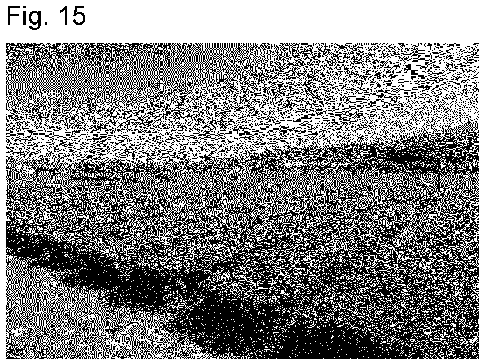

Verification test (i)
(1) 12 types of agricultural chemicals (germicides or insecticides) given below, which are used in tea leaf production fields, were each applied to approximately 3.3 m², and a total of 24 areas including A and B areas were provided (Figure 15).

A area: A mixed solution of a 50-fold dilution of the white truffle *Tuber magnatum* suspension supplemented with agricultural chemicals in a prescribed threshold quantity was applied to field tea leaves.

B area: A dilution of agricultural chemicals in a prescribed threshold quantity was applied to field tea leaves.

### [Names of agricultural chemicals used]

Acephate, difenoconazole, tebuconazole, phenthoate (PAP), methidathion, acetamiprid (neonicotinoid), silafluofen, pyriproxyfen, pyrimidifen, pirimiphosmethyl, buprofezin, and procymidone

(2) Samples were collected in constant amounts from the 12 types of application areas 5 days after application of the agricultural chemicals, and 100 g of a mixture thereof was sent to the analysis center which was requested to analyze residual agricultural chemicals. The results are as described below, and only acephate, difenoconazole, tebuconazole, PAP, and DMTP among the 12 types of agricultural chemicals were detected in both the A and B areas. The numeric values within the parentheses are the national standard values.

**[Table 8]**

| Raw tea leaf A area levels of residual agricultural chemicals in white truffle *Tuber magnatum* fermentation suspension-applied area | | |
|---|---|---|
| | Acephate: | 0.05 ppm (0.2 ppm) |
| | Difenoconazole: | 0.29 ppm (15 ppm) |
| | Tebuconazole: | 0.80 ppm (50 ppm) |
| | Phenthoate (PAP): | 0.08 ppm (0.02 ppm) |
| | Methidathion (DMTP): | 0.21 ppm (1 ppm) |

**[Table 9]**

| Raw tea leaf B area levels of residual agricultural chemicals in untreated area | | |
|---|---|---|
| | Acephate: | 0.06 ppm (0.2 ppm) |
| | Difenoconazole: | 0.28 ppm (15 ppm) |
| | Tebuconazole: | 1.00 ppm (50 ppm) |
| | Phenthoate (PAP): | 0.08 ppm (0.02 ppm) |
| | Methidathion (DMTP): | 0.35 ppm (1 ppm) |

When the numeric values of the A and B areas were compared, lower numeric values were obtained in the white truffle *Tuber magnatum* suspension-treated areas of the A areas than in spontaneous degradation. This test suggests that the application of agricultural chemicals and the white truffle *Tuber magnatum* suspension to tea leaves in a field can drastically reduce residual agricultural chemicals to below the national standard values and is therefore suitable for a raw material for the "novel tea manufacturing method" by treatment with white truffle *Tuber magnatum.*

PAP among the agricultural chemicals had a numeric value higher than the national standard both for white truffle *Tuber magnatum* and for spontaneous degradation. Thus, it is desirable to not use the agricultural chemicals PAP having residual effectiveness in tea leaf fields.

### [Experimental Example 17]

### <Verification test of novel tea manufacturing process using white truffle Tuber magnatum>

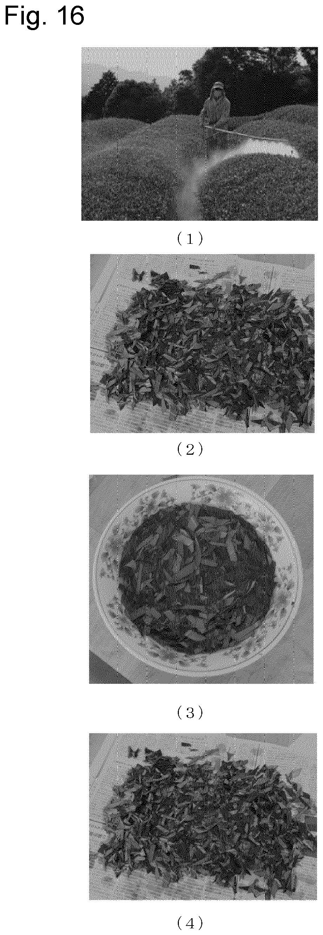

A large amount of agricultural chemicals (germicides or insecticides) is applied (usually, approximately eight times) to cultivation fields of tea leaves (Figure 16(1)), and approximately 100 types of agricultural chemicals for tea are registered and used as of 2020. As a matter of course, "tea" obtained by tea manufacturing contains "residual agricultural chemicals", albeit below the standard values, and components of the residual agricultural chemicals are naturally dissolved at the same time with components contained in tea leaves when the tea is drunk. Since tea is manufactured as a tea blended from a plurality of producers, it is almost impossible for the analysis of tea products to identify "tea plantations". This problem can be solved by inserting the step of degrading, detoxifying, and cleaning up residual agricultural chemicals to a processing step, as in processed foods. Specifically, before plucking and steaming (Figure 16(2)), tea leaves are dipped in a dilution of the white truffle *Tuber magnatum* suspension for one to several hours for cleanup (Figure 16(3)), and then washed with water (Figure 16(4)), and then, the "steaming" step can be performed.

The method of the present invention is shown in Figure 17(2). Figure 17(1) shows a conventional method.

### [Experimental Example 18]

### <Manufacturing test of immune rolled barley by use of fermentation by white truffle Tuber magnatum>

Rolled barley is utilized by people who long for health because diverse components contained in barley can be ingested by blending with white rice. However, this rolled barley also contains residual agricultural chemicals.

This rolled barley can be fermented by white truffle *Tuber magnatum* to thereby degrade and clean up diverse residual agricultural chemicals while manufacturing fermented cereal that contains pyruvic acid, choline chloride, and the like and boosts immune strength.

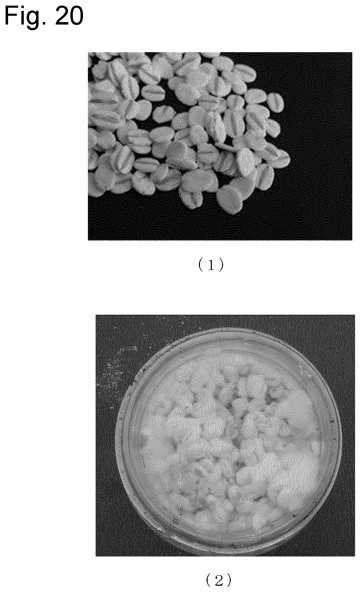

In order to ferment rolled barley (Figure 20(1)) by white truffle *Tuber magnatum,* water is added to dried rolled barley and left for 30 minutes to 2 hours so that the rolled barley sufficiently absorbs water. After discharge of extra water, 10 cc of the white truffle *Tuber magnatum* culture solution is added thereto. The rolled barley can be static-cultured in a bright place at a culture temperature from 10°C at the lowest to 15°C at the highest.

Figure 20(2) is an image 10 days after the start of culture. This fermented rolled barley can be healthy rolled barley containing components such as pyruvic acid, a component produced by white truffle *Tuber magnatum.* The same holds true for cereal other than rolled barley. White truffle *Tuber magnatum* produces pyruvic acid from starch through saccharification and the glycolysis pathway. Although fermentation by koji mold produces glucose through saccharification, any fermenter that can produce pyruvic acid has not been known.

### [Experimental Example 19]

### <Manufacturing test of immune jam by use of fermentation by white truffle Tuber magnatum>

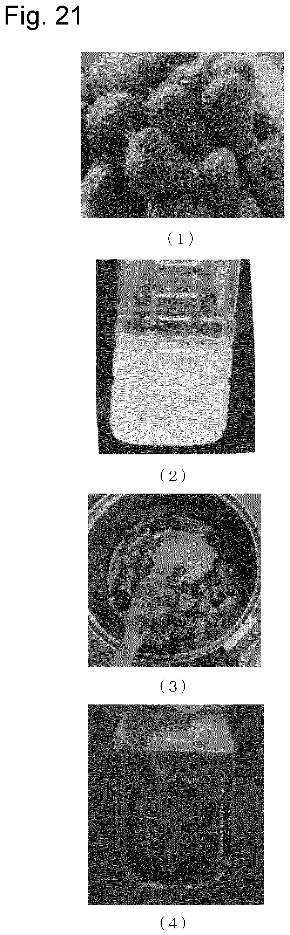

Strawberry (Figure 21(1)) for use as a raw material is dipped in a 30-fold dilution of the white truffle *Tuber magnatum* suspension in advance to degrade and clean up residual agricultural chemicals. Residual agricultural chemical-free pyruvic acid-containing novel strawberry jam can thereby be manufactured. Furthermore, the white truffle *Tuber magnatum* culture solution of the present invention is a strongly acidic solution of pH 4.0 having the ability to produce pyruvic acid (Figure 21(2)) and as such, can be used as an acidic agent for pectin gelling. When table sugar is added and concentrated in a basic process of strawberry jam manufacturing, the white truffle *Tuber magnatum* culture solution is appropriately added for pectin gelling to prepare a gel. Figure 21(1) shows strawberry jam completed by bottle filling and heat sterilization.

Vinegar, lemon juice, or the like is generally used for pectin gelling. In the present invention, immune jam containing pyruvic acid and the like can be prepared by use of the white truffle *Tuber magnatum* culture solution. Jam may be manufactured by the same method as above using other fruits.

### [Experimental Example 20]

### <Short-time manufacturing test of immune fruit juice pyruvic acid beverage by use of fermentation by white truffle Tuber magnatum>

Use of fermentation by white truffle *Tuber magnatum* enables a novel "immune fruit juice" "beverage" to be manufactured in a short time, which is impossible for previous fermentation, and can drastically reduce manufacturing cost.

According to the present invention, a novel "immune fruit juice" "beverage" can be manufactured from almost all of fruits (leaves, stems, and rhizomes) because the fruits can be fermented in approximately 3 to 5 days by fermentation by white truffle *Tuber magnatum* as long as containing carbohydrate even slightly.

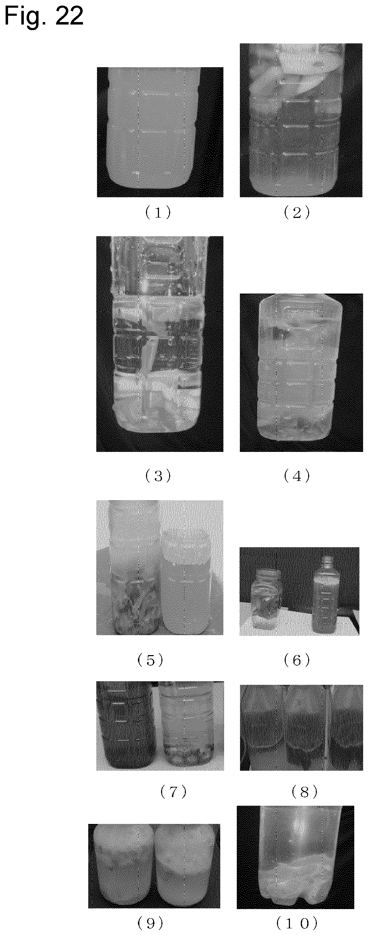

Figure 22 (1) shows a state 4 days after the start of culture of banana (1), chocolate vine (2), persimmon (sweet persimmon) (3), tomato (4), apple (5), peach (6), blueberry (7), watermelon (8), mango (9), and mandarin orange (10).

### Testing method

10 cc of the white truffle *Tuber magnatum* culture solution is added to 1000 cc of water and 200 g of fruit slices or 200 cc of ground fruit juice, followed by static culture under aerobic conditions involving a culture temperature from 10°C at the lowest to 25°C at the highest. Since strong antimicrobial activity of white truffle *Tuber magnatum* inactivates microbes attached to the raw material, it is not necessary to wash the raw material fruit with water or to heat-sterilize the fruit, for example.

White truffle *Tuber magnatum* ultrarapidly degrades lignin in the plant, leaches components out, and ultrarapidly ferments the components. Therefore, the fermentation completes in 3 days of culture.

Thus, according to the present invention, a physiologically active substance or a bioactive substance in a fruit can be extracted in 3 days of culture to manufacture a novel immune fruit juice beverage supplemented with pyruvic acid, a component fermented by white truffle *Tuber magnatum.* Then, the fermentation is inactivated by heat sterilization at 100°C for approximately 10 minutes. The fermentation by white truffle *Tuber magnatum* produces no alcohol and therefore enables a "non-alcoholic" beverage to be manufactured.

Also, fruit juice beverages may be blended according to preference.

Further, a "carb-free" immune beverage can be produced because the fermentation by white truffle *Tuber magnatum* rapidly degrades carbohydrate and glucide and modifies them into pyruvic acid.

### [Experimental Example 21]

### <Manufacturing test of pyruvic acid-containing chocolate by use of fermentation by white truffle Tuber magnatum>

Pyruvic acid can be easily manufactured by fermenting various plant raw materials by the method of the present invention. Therefore, a pyruvic acid-containing fermentation solution can be added to various foods either directly or as a powder after being dehydrated and dried.

Chocolate is currently consumed as a preferred food. Such chocolate is allowed to contain and carry pyruvic acid and is thereby capable of becoming a totally different product, i.e., next-generation chocolate allowed to carry an immunity-boosting function or chocolate for recovery from fatigue or prevention of fatigue.

### [Manufacturing Example]

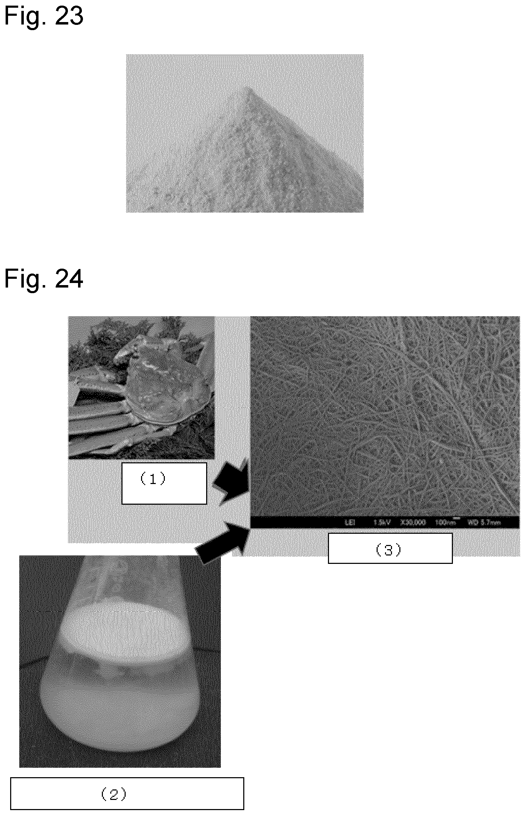

A chocolate raw material (Figure 23(1)) is crushed and kneaded, and the chocolate raw material powder is kneaded into a mash (Figure 23(2)).

This mash is mixed with approximately 10% of a dried banana powder fermented by white truffle *Tuber magnatum* to complete banana pyruvic acid-containing chocolate (Figure 23(3)).

### [Experimental Example 22]

### <Manufacturing test of functional immune bread containing pyruvic acid and mycelium-derived chitin nanofiber by use of fermentation by white truffle Tuber magnatum>

Successful mass culture of white truffle *Tuber magnatum* mycelium enables chitin and chitin nanofiber to be easily manufactured from the mycelium (Figures 24(2) and 24(3)). Chitin and chitin nanofiber are currently manufactured from "crab shell" (Figure 24(1)) and however, cannot be manufactured in landlocked countries. By contrast, white truffle *Tuber magnatum* mycelium can be cultured anywhere and therefore allows for immune strength boosting and rapid energy supply throughout the world, and further enables bread improved in texture to be manufactured by the addition thereof to the bread.

### [Manufacturing Example]

A heat-sterilized 30-fold dilution of the white truffle *Tuber magnatum* culture solution is added instead of water to bread flour to prepare pyruvic acid-containing bread dough, which is then fermented by the addition of yeast to manufacture bread.

This 30-fold dilution of the white truffle *Tuber magnatum* culture solution is rich in pyruvic acid and however, may have a small content of chitin fiber. In such a case, a chitin fiber powder is appropriately added thereto for use.

Bread having fluffy and puffy texture can thereby be prepared in which particles of bread flour are linked and bridged through nanofiber. Such bread is allowed to contain pyruvic acid and can thereby be manufactured as immune functional bread having fluffy texture, which has been impossible so far.

### [Experimental Example 23]

### <Manufacturing test of diverse ice cream-related products supplemented with pyruvic acid-containing fruit juice by use of fermentation by white truffle Tuber magnatum>

Current ice cream-related products are manufactured, sold, and consumed as preferred foods. Such products are supplemented with fruit juice fermented by white truffle *Tuber magnatum* with banana or the like as a raw material, and can thereby become products carrying a novel immunity-boosting, summer fatigue-preventing, or energy-replenishing function, despite being preferred foods.

The addition of pyruvic acid-containing fruit juice fermented by white truffle *Tuber magnatum* enables diverse fruit-flavored products to be prepared. Furthermore, a more complicated immune functional ice cream-related product containing pyruvic acid and lactic acid bacteria can be manufactured by mixing with a dairy product or a lactic acid bacterium-fermented solution.

### [Experimental Example 24]

### <Manufacturing test of pyruvic acid-containing immunity-boosting noodle by fermentation by white truffle Tuber magnatum>

Japanese often eat noodles. Such noodles are allowed to contain pyruvic acid and can thereby be manufactured as immune strength-boosting or energy-supplying or -complementing next-generation noodles.

### [Manufacturing method]

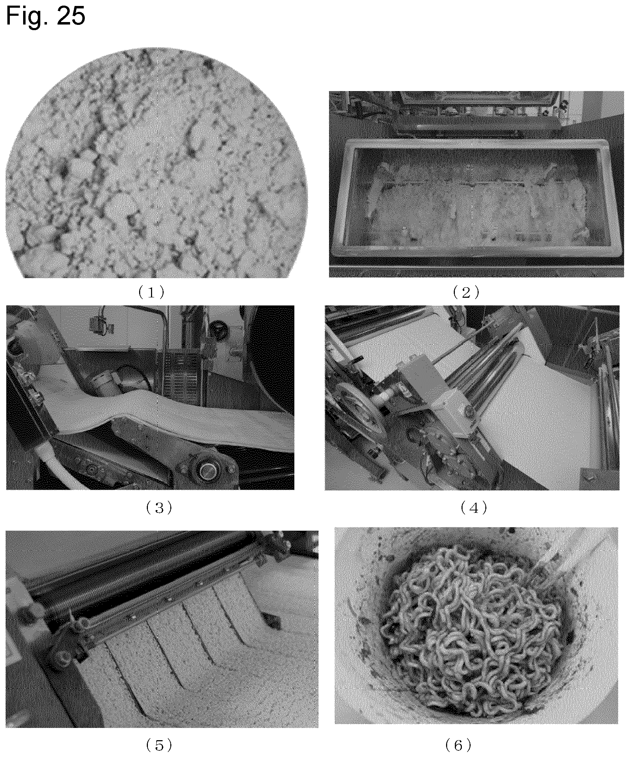

Figure 25(1) shows kneading water adjustment: a state supplemented with 1 kg of wheat, 10 to 30 g of salt, and brine.
Figure 25(2) shows mixing: a state kneaded at a liquid temperature of 20 to 30°C for 15 to 20 minutes after addition of 300 to 400 cc of an aqueous suspension fermented by white truffle *Tuber magnatum.*
Figure 25(3) shows a noodle sheet: a sheet form is prepared.
Figure 25(4) shows rolling: the step of applying pressure to the noodle sheet into a thin sheet.
Figure 25(5) shows cutting into strips: cutting.
Figure 25(6) shows completed pyruvic acid-containing noodles.

### [Experimental Example 25]

### <Manufacturing test of araliaceous plant green juice by use of fermentation by white truffle Tuber magnatum>

Diverse green juice to complement a lack of vegetables, lactic acid bacterium-containing green juice aimed at intestinal regulation, and the like are currently manufactured and sold. In the present invention, short-time fermentation by white truffle *Tuber magnatum* weakens cells and thereby improves digestion and absorption in the intestine, while a residual agricultural chemical-free pyruvic acid-containing immune strength-boosting or energy-replenishing green juice as well as multifunctional green juice further inhabited by lactic acid bacteria so as to have intestinal regulation action can be manufactured by fermentation by white truffle *Tuber magnatum.*

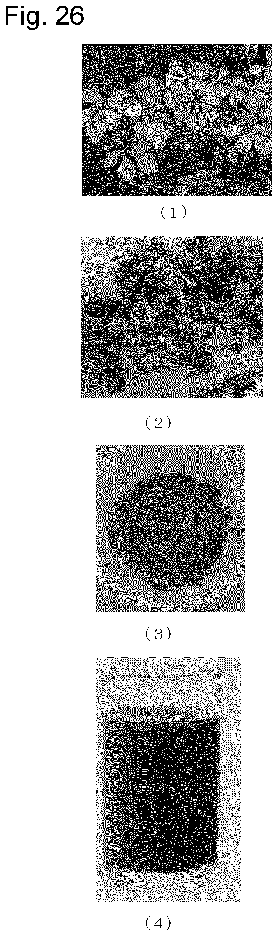

This test was conducted using, as a raw material, "araliaceous plant" (Figures 26(1) and 26(2)) which is eaten as a completely chemical-free spring mountain vegetable produced in Yamagata, Japan.

In this test, this araliaceous plant is fermented by white truffle *Tuber magnatum* for several days, freeze-dried in vacuum, and processed into a powder (Figure 26(3)). This powder is dissolved in water or hot water and can thereby be relished (Figure 26(4)).

### [Experimental Example 26]

### <Manufacturing test of functional immune food by combination of lactic acid bacterium-fermented food and white truffle Tuber magnatum-fermented food>

Processed foods are mostly fermented using lactic acid bacteria, koji mold, or yeast. Novel pyruvic acid-containing diverse immune functional fermented products can be manufactured by adding pyruvic acid, chitin nanofiber, or other fermentation product components of a pear solution fermented by white truffle *Tuber magnatum* to miso, soy sauce, a lactic acid bacterium-fermented product, pickles, soymilk, or the like.

### [Manufacturing Example 1]

Pyruvic acid-containing and chitin nanofiber-containing immune functional miso can be manufactured by adding a pear solution (heat-sterilized) fermented by white truffle *Tuber magnatum* to miso.

The pear solution fermented by white truffle *Tuber magnatum* has pH 4.0 and however, has no sourness and therefore does not impair the taste or flavor of miso.

### [Manufacturing Example 2]

Pyruvic acid-containing and chitin nanofiber-containing immune functional soy sauce can be manufactured by adding a pear solution (heat-sterilized) fermented by white truffle *Tuber magnatum* to soy sauce. In this case as well, the taste or flavor of soy sauce is not impaired.

### [Manufacturing Example 3]

Pyruvic acid-containing and chitin nanofiber-containing immune functional yogurt can be manufactured by adding a pear solution (heat-sterilized) fermented by white truffle *Tuber magnatum* to yogurt.

### [Manufacturing Example 4]

Pyruvic acid-containing and chitin nanofiber-containing immune functional pickles can be manufactured by adding a pear solution (heat-sterilized) fermented by white truffle *Tuber magnatum* to various pickles.

### [Experimental Example 27]

### <Manufacturing test of livestock feed by fermentation by white truffle Tuber magnatum>

The amount of beef consumed is particularly increasing in economically developed countries. Most parts of bovine, pig, or chicken feed are composed of cereal which is a human food. A future food shortage immediately leads to reduction in production of meat, egg, milk, and the like. The boosting of feed efficiency is an innovative new technique in the farming industry.

Currently, the eating of 10 kg of cereal results in the production of 1 kg of meat. Protein ingestion from meat is the waste of foods. Accordingly, far-seeing research on "artificial meat" has been started. The present inventor intends to cope with this by developing a technique of boosting feed efficiency.

Enriched feed for livestock is cereal, cereal residues, or the like, which is currently used as feed directly. Such feed is fermented by white truffle *Tuber magnatum* and can thereby be modified into immune feed containing diverse components such as pyruvic acid. This can boost feed efficiency. Wild herbivores, when preying on wild grass, eat soil and wild grass caked with soil at the same time, on which white wood-rotting fungi that degrade withered leaves grow and adhere. That is, wild herbivores eat white wood-rotting fungi. Their lignin, cellulose-degrading enzyme, lignin peroxidase, and manganese peroxidase are taken up into the body so that persistent plant fiber is degraded. Fermented feed in preceding findings in previous farming includes silage fermented by lactic acid bacteria. The lactic acid bacteria are bacteria and can degrade neither lignin nor cellulose. In the preceding findings, white wood-rotting fungi have been eliminated or ignored. By contrast, the present invention is directed to the reproduction of wild prey containing white wood-rotting fungi for herbivores.

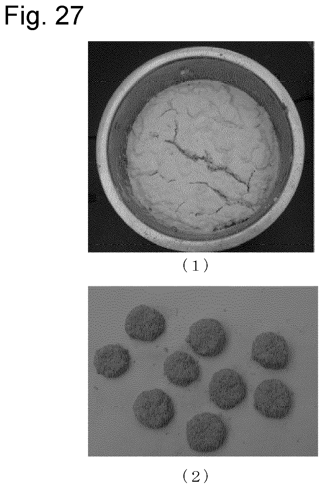

Rice bran (500 g) + wheat bran (500 g) is used as a test material, and its humidity is adjusted to 80% by appropriate addition of water. 10 cc of the white truffle *Tuber magnatum* culture solution was added thereto, followed by static culture with a thickness of approximately 50 cm at a temperature from 5°C at the lowest to 20°C at the highest for 7 days under aerobic conditions (Figure 27(1)). The resultant is processed into granules, heat-dried for the inactivation of white truffle *Tuber magnatum,* and applied to feed (Figure 27(2)).

Not only feed for livestock but pyruvic acid-containing energy-complementing health foods for immunity boostings, antiagings, and long healthy lives which are different from previous rice bran health foods can be prepared by changing materials other than rice bran.

### [Experimental Example 28]

### <Test of livestock feed by fermentation by white truffle Tuber magnatum>

A basic test was carried out on whether livestock would be willing to eat feed fermented by white truffle *Tuber magnatum.* This is because such feed cannot be given every day unless livestock is willing to eat it.

Accordingly, immune pyruvic acid-containing functional enriched feed of rice bran fermented by white truffle *Tuber magnatum* was manufactured as follows.

Rice bran having a humidity adjusted to 80% (humidity of Oshibori (hot wet towel)) by appropriate addition of water was appropriately supplemented with an undiluted liquid of white truffle *Tuber magnatum* with a thickness of approximately 5 to 10 cm, and fermentation-cultured at a temperature from 5 to 20°C at the lowest to 20 to 50°C at the highest for 2 to 5 days under aerobic conditions.

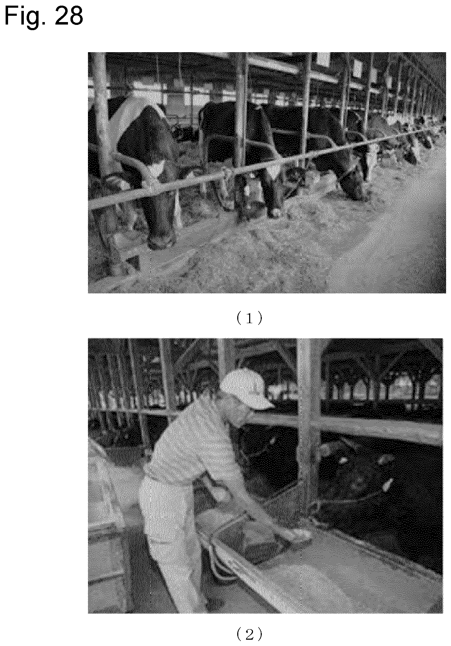

The feed thus manufactured was sprinkled to hay, and cows were allowed to eat it (Figure 28(1)). Further, fattened Japanese cattle were allowed to eat the feed alone (Figure 28(2)).

The feed was fiercely eaten in all the cases and thus found to be excellent in preference.

### [Experimental Example 29]

### <Livestock feed and chicken egg production test by fermentation by white truffle Tuber magnatum>

Immune pyruvic acid-containing functional enriched feed of rice bran fermented by white truffle *Tuber magnatum* was manufactured.

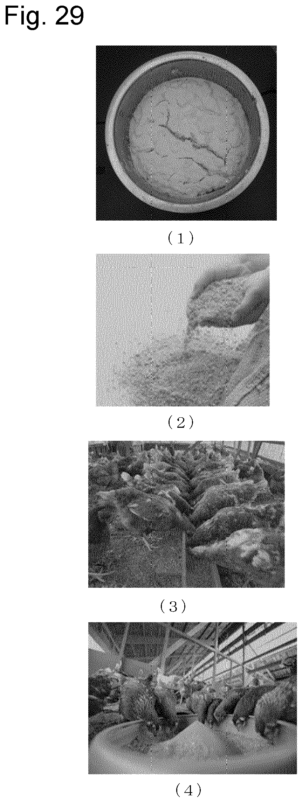

Rice bran having a humidity adjusted to 80% (humidity of Oshibori (hot wet towel)) by appropriate addition of water was adjusted to a rice bran thickness of approximately 5 to 10 cm by appropriate addition of an undiluted liquid of white truffle *Tuber magnatum,* and fermentation-cultured at a temperature from 5 to 20°C at the lowest to 20 to 50°C at the highest for 2 to 5 days under aerobic conditions (Figure 29(1)). The resultant was dried (Figure 29(2)) and given to Nagoya Cochin either alone (Figure 29(3)) or together with another feed (Figure 29(4)).

### [Experimental Example 30]

### <Short-time extraction test of physiologically active substance or bioactive substance by fermentation by white truffle Tuber magnatum>

White truffle *Tuber magnatum* has been adopted to the present invention because white truffle *Tuber magnatum,* albeit ascomycetous, possesses characteristics of white wood-rotting fungi, and can degrade and weaken lignin and cellulose in cells of leaves, flowers, fruits, stems, roots, tuberous roots, bulbs, and the like in plant tissues and thereby elute all intracellular components to the outside of the cells in a short time, which is impossible for other fermentative microbes, while this test can manufacture an agricultural chemical-free pyruvic acid-containing beverage by performing degrading and cleaning up residual agricultural chemicals in a raw material at the same time. Agricultural chemical-free pyruvic acid-containing fruit juice and herbal medicine beverage were manufactured in a short time by use of such characteristics. Use of fermentation by white truffle *Tuber magnatum* enables most components to be extracted in 3 to 5 days from most plant raw materials, and enables an herb-, medicinal plant-, or Chinese medicine-containing beverage to be manufactured in a short time.

### [Manufacturing Example]

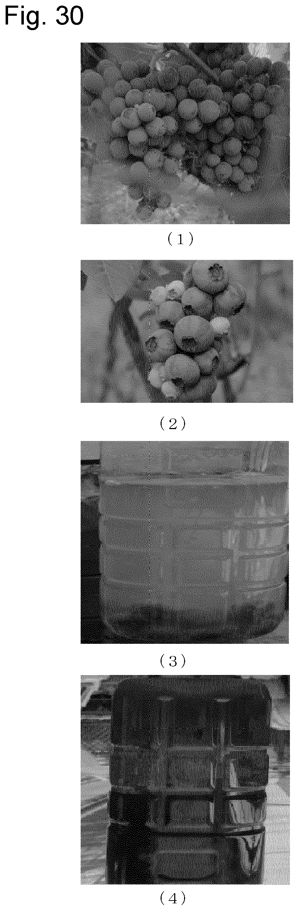

Grape (black grape) (Figure 30(1)) and blueberry (Figure 30(2)) were used as raw materials. 100 g of each raw material was washed with water and added to 500 cc of water, and 10 cc of an undiluted liquid of white truffle *Tuber magnatum* was added thereto (Figure 30(3)).

Static culture was performed in a bright place at a culture temperature from 10°C at the lowest to 25°C at the highest

All intracellular components such as polyphenol were leached out as a result of degrading and weakening lignin and cellulose in the cells of each raw material 7 days after the start of culture (Figure 30(4)).

### [Experimental Example 31]

### <Persimmon tannin extraction test by fermentation by white truffle Tuber magnatum>

Previous persimmon tannin manufacturing methods require approximately 2 to 3 years because green persimmon (immature fruit) of astringent persimmon is fermented by yeast for extraction. Further oxidation yields a "brown" solution. According to the present invention, persimmon tannin is extracted in approximately 7 to 10 days by novel fermentation by an ascomycetous white wood-rotting fungus. Furthermore, oxidation can be inhibited by antioxidative enzyme of white truffle *Tuber magnatum* to manufacture a "clear colorless persimmon tannin solution".

Although diverse "tea" is currently manufactured from many plants, most of the products contain a plant physiologically active substance tannin. White truffle *Tuber magnatum* contains an enzyme inhibiting oxidation, and therefore allows for manufacturing of an oxidative browning-free persimmon tannin solution and other solutions, such as tea, containing tannin.

### [Manufacturing Example]

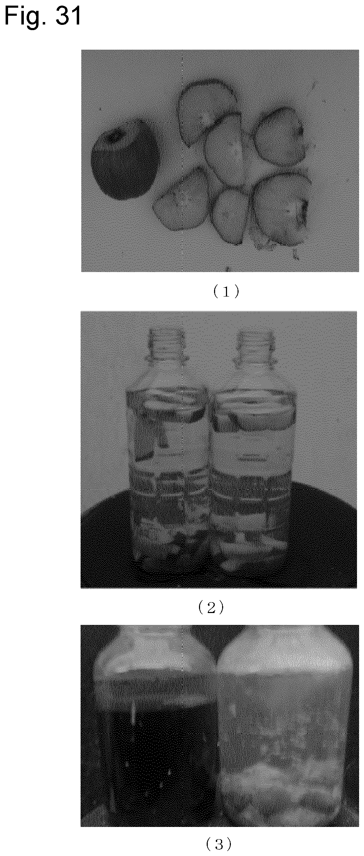

Green persimmon or semimature fruit of cultivar name "Gosho Persimmon" (astringent persimmon produced in Yamagata, Japan) was used as a raw material. 50 g of the astringent persimmon was washed with water, then appropriately cut (Figure 31(1)), and added to 500 cc of water, and the mixture was added to a 500 cc PET bottle, to which 10 cc of an undiluted liquid of white truffle *Tuber magnatum* was then added, followed by fermentation by white truffle *Tuber magnatum* for approximately 7 to 15 days under temperature conditions from 5°C at the lowest to 20°C at the highest. A "transparent persimmon tannin solution" was completed 15 days after fermentation (Figure 31(2)).

This "transparent persimmon tannin solution" was analyzed at "Japan Food Research Laboratories" and consequently contained 0.05 g of tannin (as tannic acid) in 100 g.

On the other hand, in the case of extraction by a conventional yeast fermentation method instead of white truffle *Tuber magnatum,* the solution is oxidated and thereby discolored into brown by the action of polyoxy oxidative enzyme. The same experiment as above was conducted for yeast and white truffle *Tuber magnatum,* and both the results were compared. Figure 31(3)) shows a persimmon tannin solution during manufacturing by yeast fermentation (left) and a persimmon tannin solution during manufacturing by fermentation by white truffle *Tuber magnatum* (right), the difference between which is evident.

In the case of manufacturing a processed food or a beverage, this "discoloration" ascribable to polyoxy oxidation is a huge problem. The inhibition of this problem by fermentation by white truffle *Tuber magnatum* is capable of simplifying a manufacturing process and reducing manufacturing cost.

### [Experimental Example 32]

### <Extraction test of wasabi bioactive component allyl isothiocyanate by fermentation by white truffle Tuber magnatum>

Wasabi of the family *Brassicaceae* contains a physiologically active substance allyl isothiocyanate as antimicrobial and anti-insect measures. Wasabi has been continuously used in "sushi" and the like by use of such characteristics.

This test was carried out to extract allyl isothiocyanate by fermentation by white truffle Tuber *magnatum.*

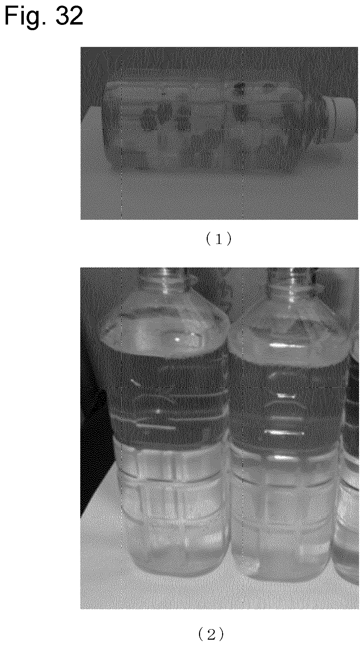

Wasabi rhizomes sliced into 1 cm were placed in a 500 cc PET bottle filled with 500 cc of water, and 10 cc of an undiluted liquid of white truffle *Tuber magnatum* was added thereto, followed by culture at a temperature from 10°C at the lowest to 20°C at the highest for 3 to 5 days (Figure 32(1)).

Then, the extracted solution was filtered and heat-sterilized at 100°C for 5 minutes to obtain an extracted allyl isothiocyanate solution (Figure 32(2)).

### [Experimental Example 33]

### <Extraction test of pine needle active component α-pinene by fermentation by white truffle Tuber magnatum>

α-Pinene possesses antimicrobial and anti-insect characteristics and is abundantly contained in pine, mioga ginger, and the like. A healing component that enters the nasal cavity first in exposure to forests is derived from the odor of α-pinene. This test employed pine needle for the purpose of low-cost extraction by fermentation by white truffle *Tuber magnatum,* and an insecticidal or repellant test of aphids by an extracted solution was further conducted at the same time therewith in order to verify the effect thereof.

### [Testing method]

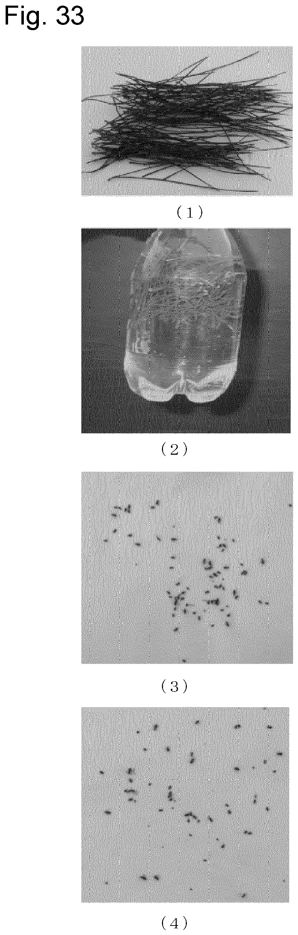

100 g of black pine needle (Figure 33(1)) was placed in a PET bottle containing 500 cc of water, and 10 cc of an undiluted liquid of white truffle *Tuber magnatum* was further added thereto, followed by static culture at a temperature from 10°C at the lowest to 25°C at the highest.

In order to confirm that α-pinene was extracted into the culture solution on the 5th day of fermentation by white truffle *Tuber magnatum* (Figure 33(2)), a 10-fold dilution was sprayed to aphids that actively moved around (Figure 33(3)). All the aphids no longer moved and died 12 hours after spraying (Figure 33(4)).

### [Experimental Example 34]

### <Degradation test of plant waste oil by fermentation by white truffle Tuber magnatum>

Plant waste oils from food processing or restaurants will become heavy industrial garbage in the future (due to the abolition of diesel vehicles where such plant waste oils are recycled). Use of white truffle *Tuber magnatum* in the treatment thereof allows for degradation treatment in a short time.

2 cc of white truffle *Tuber magnatum* was added to 100 cc of a waste oil of rapeseed oil. Figure 34 shows a state 7 days later at ordinary temperature. As is evident from the photograph, white truffle *Tuber magnatum* vigorously propagates by preying on the waste oil.

### [Experimental Example 35]

### <Manufacturing test of material for reduced chemical fertilizer and reduced chemical and chemical-free cultivation by treatment of processed food residue by use of fermentation by white truffle Tuber magnatum>

A main object of the present invention is to perform permanent stable production of sustainable agricultural chemical-free processed food raw materials in fields throughout the world by preparing materials for reduced chemical fertilizer and reduced chemical and chemical-free cultivation by the fermentation of "downstream" food processing residues or waste oils of processed foods by white truffle *Tuber magnatum.*

White truffle *Tuber magnatum* is applied to a banana residue (Figure 35(1)), which is generated during manufacturing of processed foods, so that the banana residue is fermented and degraded by the white truffle *Tuber magnatum* and thereby modified into a material for chemical-free cultivation (Figure 35(2)).

This material is applied to a field so that the mycorrhizal fungus white truffle *Tuber magnatum* cohabits in the soil. Therefore, stable growth and production of crops can be expected even under changes in climate.

### [Experimental Example 36]

### <Plant tissue permeability test of white truffle Tuber magnatum culture suspension>

In the present invention, a processed food or beverage raw material is dipped in a dilution of the white truffle *Tuber magnatum* suspension to degrade, detoxify, and clean up residual agricultural chemicals in the raw material by the degradation ability of the white wood-rotting fungus. For this purpose, degrading enzyme of white truffle *Tuber magnatum* needs to rapidly penetrate cells of the raw material. This test is a test on the penetration rate.

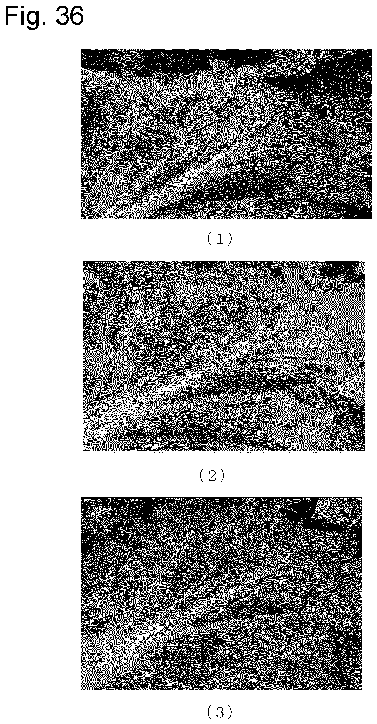

Chinese cabbage leaves were used as a test material, and a 30-fold dilution of the white truffle *Tuber magnatum* suspension was applied thereto under conditions involving a temperature of 15°C and a humidity of 90% (Figure 36(1)).

The dilution of white truffle *Tuber magnatum* started to penetrate leaf cells 5 minutes after application (Figure 36(2)) and was almost absent on the surface of the leaves 10 minutes thereafter while white truffle *Tuber magnatum* penetrated the leaf cells (Figure 36(3)).

### [Experimental Example 37]

### <Plant hormone production test by fermentation of processed food residue by white truffle Tuber magnatum>

In the case of treating a processed food residue by fermentation by white truffle *Tuber magnatum,* and thereby modifying the residue into a material for reduced fertilizer and reduced chemical and chemical-free cultivation, large amounts of a plant hormone indole-3-acetic acid and an energy source pyruvic acid are secondarily produced as by-products into the resulting fermentation solution. Mycorrhizal fungal hypha of white truffle *Tuber magnatum* supplies this plant hormone and pyruvic acid to a plant. As a result, an excellent material for reduced fertilizer and reduced chemical and chemical-free cultivation is obtained owing to the energy of the pyruvic acid, the plant hormone which promotes cell division, and the antimicrobial activity of white truffle *Tuber magnatum.*

The test of Experimental Example 4 verified that white truffle *Tuber magnatum* is a very rare fungus that produces the plant hormone indole-3-acetic acid. For stable food production under changes in climate, many researchers around the world are currently searching for a microbe that produces a plant hormone produced by all plants in their bodies. Indole-3-acetic acid is also necessary for cell proliferation in human bodies and is an essential component for the turnover of intestinal cells and the proliferation of immunocytes. Therefore, white truffle *Tuber magnatum* which is producible at low cost will presumably become very important in the future.

In order to verify the effect of the plant hormone produced by white truffle *Tuber magnatum,* this test was conducted to perform the fertilizer-free cultivation of rice and the cultivation of dianthus using the plant hormone-containing fermentation solution obtained in the test of Experimental Example 4.

### [Fertilizer-free cultivation of rice]

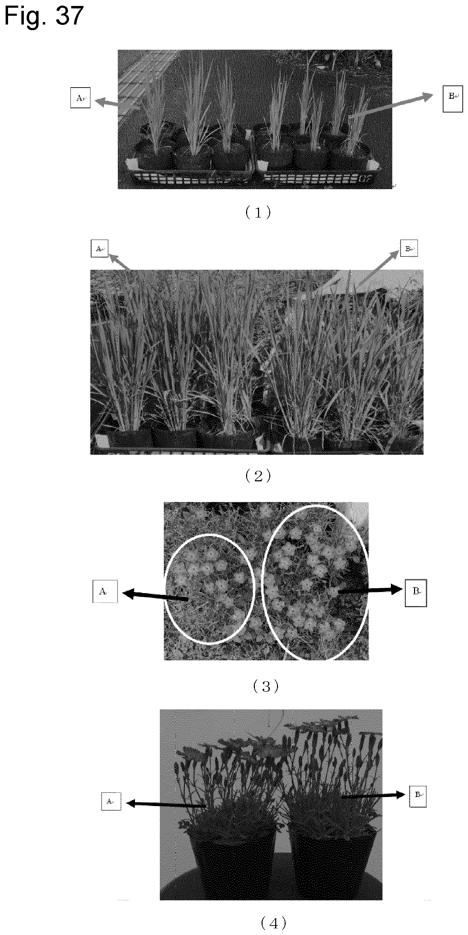

Rice seedlings to which 10-fold dilution of the plant hormone was applied (A) and unapplied rice seedlings (B) were provided (Figure 37(1)), and the growth state of rice was observed 1 month later (Figure 37(2)).

As a result, in the plant hormone-applied area (A) compared with the unapplied area (B), the plant length was higher by approximately 20%, and the number of stems was also increased, showing growth leading to high yields.

### [Cultivation of dianthus]

Two pots of container-grow dianthus were provided, and a 10-fold dilution of the plant hormone was applied to one of the pots twice at a 10-day interval. The untreated pot was designated as (A). The plant hormone-applied pot was designated as (B).

Figure 37(3) shows the blooming status of dianthus 26 days later. The number of flowers was larger in the plant hormone-applied pot (B). Figure 37(4) is an image taken edge-on. In the plant hormone-applied pot (B), the plant length was higher by approximately 20%, and the plant grew into a large size because of larger propagation of the plant foot.

### [Experimental Example 38]

### <Degradation and cleanup test of honey neonicotinoid by white truffle Tuber magnatum>

Honey has been very often used as an additive for health foods and processed foods, and imported from the world according to growing demand. Such honey may contain an insecticide neonicotinoid.

In recent years, residual agricultural chemicals contained in honey have become a serious problem worldwide. Particularly, from the finding that neonicotinoid-based insecticides destroy the honeybee's ecosystem, absolute ban on the use thereof has been carried out in European countries and the like. By contrast, in Japan, residual standard values have rather been drastically raised.

However, honey production sites and consumers are seriously concerned about honey containing residual agricultural chemicals, and there is growing longing for safe and secure honey. Accordingly, the presence or absence of degradation of a honey-containing neonicotinoid-based insecticide by white truffle *Tuber magnatum* was tested.

### Photograph 1

Left: MM2 area Honey mixed with the white truffle *Tuber magnatum* suspension
Right: MG1 area Untreated area

### [Testing method]

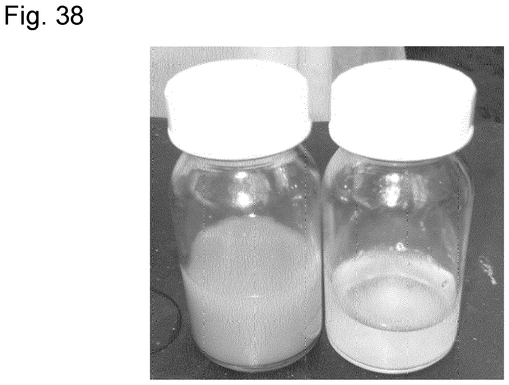

100 cc of honey was used as an untreated area (MG1), and 10 cc of the white truffle *Tuber magnatum* suspension was added to 100 cc of honey, stirred, and then left in a room at ordinary temperature for 5 days to prepare a white truffle *Tuber* magnatum-treated area (MM2) (Figure 38). Honey samples from these two areas were sent to an analytics company (Mie Prefecture Environmental Conservation Agency), and analyzed and assayed for a residual insecticide.
MM2 (left in the photograph): 0.01 ppm (detection limit)
MG1 (right in the photograph): 0.03 ppm

The residual concentration of the neonicotinoid-based insecticide nitenpyram was decreased in the MM2 area compared with the untreated area. This suggests that white truffle *Tuber magnatum* caused degradation and cleanup to progress in 5 days of treatment. Although October is a season when the activity of white truffle *Tuber magnatum* is reduced at room temperature, degradation was started, suggesting that 20 to 30 days are required for larger degradation.

### Industrial Applicability

The present inventor has found for the first time in the world that white truffle *Tuber magnatum* produces such physiologically active substances and is also the most important fermenter that is harmless to humans and animals. The present inventor has thereby developed a novel innovative fermentation technique that can manufacture a revolutionary immunity-boosting or energy-replenishing or -complementing product. The ascomycetous white wood-rotting fungus newly added to the fermentation world allows for manufacturing of a fermented product containing pyruvic acid and the like, which has been absent throughout recorded history. Furthermore, the new finding about fermentation by white truffle *Tuber magnatum* which is the ascomycetous white wood-rotting fungus of the present invention allows for manufacturing of diverse pyruvic acid-containing fermented foods, processed foods, beverages, tea, herbal medicines, and livestock feed products for immunity boostings, antiagings, health maintenances, and long healthy lives adapted to cleanup of residual agricultural chemicals, the post-corona society, the decarbonized society, coexistence with viruses, the immune society, and the super-aging society, which are impossible to manufacture by fermentation by lactic acid bacteria, yeast, koji mold, or *Bacillus subtilis* var. *natto.* Moreover, hydrogen can be produced from a food processing residue by combining the method of the present invention with the modification of the processed food residue into a material for reduced fertilizer and reduced chemical and chemical-free cultivation by treatment that generates no food processing garbage, or fermentation by an anaerobic hydrogen-producing bacterium. Besides, a plant physiologically active substance or bioactive substance can be extracted in a short time by use of the wood-rotting fungal characteristics of white truffle *Tuber magnatum.* A secondarily produced plant hormone indole-3-acetic acid which achieves promoted and stable crop growth under changes in climate is a highly notable fermentation product and offers a new realm to the world. The present invention enriches life in a new lifestyle, economy, diet, and agriculture by use of products in more diverse regions than those of previous fermented foods and residue treatment through the innovation of the food, beverage, and livestock feed industries by the novel innovative novel fermentation technique.

## Claims

1. A method for fermenting and degrading a compound by an ascomycetous white wood-rotting fungus having mycorrhizal characteristics.

2. A method for manufacturing a fermented product by use of fermentation and degradation according to claim 1.

3. The method according to claim 1 or 2, wherein the ascomycetous white wood-rotting fungus having mycorrhizal characteristics is white truffle *Tuber magnatum.*

4. A method for degrading, detoxifying, and cleaning up residual agricultural chemicals in a food raw material by use of fermentation and degradation according to claim 1.

5. A method for producing pyruvic acid, choline chloride, butanediol, indole-3-acetic acid, or 3-hydroxybutyric acid by fermentation and degradation according to claim 1.

6. A method for manufacturing a processed food, a beverage, tea, an herbal medicine, livestock feed, or other products containing pyruvic acid, choline chloride, butanediol, indole-3-acetic acid, or 3-hydroxybutyric acid by use of a method according to claim 5.

7. A method for extracting a plant physiologically active substance by use of fermentation and degradation according to claim 1.
